(19)

**Europäisches Patentamt**

**European Patent Office**

**Office européen des brevets**

(11) **EP 4 685 469 A1**

(12) **EUROPEAN PATENT APPLICATION**

(43) Date of publication:
28.01.2026 Bulletin 2026/05

(21) Application number: 24190422.6

(22) Date of filing: 23.07.2024

(51) International Patent Classification (IPC):
*G01N 21/85* (2006.01)    *G01N 21/359* (2014.01)
*G01N 21/3563* (2014.01)    *G01N 21/64* (2006.01)
*G01N 21/65* (2006.01)    *B03C 7/00* (2006.01)
*G01N 33/10* (2006.01)    *G01N 21/84* (2006.01)
*G01N 15/00* (2024.01)    *G01N 1/00* (2006.01)

(52) Cooperative Patent Classification (CPC):
**G01N 21/85; B03C 7/006; G01N 21/3563;
G01N 21/359; G01N 21/645; G01N 21/6486;
G01N 21/65; G01N 33/10;** G01N 2015/0019;
G01N 2021/8416; G01N 2021/8466;
G01N 2021/8592; G01N 2201/0221;
G01N 2201/129

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
**BA**
Designated Validation States:
**GE KH MA MD TN**

(71) Applicant: **Mühlenchemie GmbH & Co. KG**
**22926 Ahrensburg (DE)**

(72) Inventors:
• **Ziegler, Denise Sarah Tanja**
**70469 Stuttgart (DE)**

• **Hitzmann, Bernd**
**30559 Hannover (DE)**
• **Popper, Lutz**
**22926 Ahrensburg (DE)**
• **Nåbo, Edvin**
**22926 Ahrensburg (DE)**
• **Zhao, Chenxi**
**22041 Hamburg (DE)**

(74) Representative: **Moré, Solveig Helga et al**
**Kroher Strobel**
**Rechts- und Patentanwälte PartmbB**
**Bavariaring 20**
**80336 München (DE)**

(54) **METHODS AND DEVICES FOR PREDICTING THE PROCESSING PROPERTIES OF WHEAT FLOUR**

(57)    The present invention relates to the field of food science. In particular, the present invention provides a method for predicting at least one processing property of a ground cereal product, e.g. a wheat flour, comprising steps of separating the ground cereal product into at least two distinct fractions, analysing at least one of these fractions by spectroscopy, and on the basis of the analysis, predicting the at least one processing property of interest of the ground cereal product using a mathematical model. The at least one processing property of interest may be a baking property or a rheological property of dough prepared from the ground cereal product. The present invention further provides a portable device for the preparation and spectroscopic analysis of grain samples suitable for performing the prediction method of the invention, and a software product.

**Fig. 1**

EP 4 685 469 A1

**Description**

The present invention relates to the field of food science. In particular, the present invention provides a method for predicting at least one processing property of a ground cereal product, e.g. a wheat flour, comprising steps of separating the ground cereal product into at least two distinct fractions, analysing at least one of these fractions by spectroscopy, and on the basis of the analysis, predicting the at least one processing property of interest of the ground cereal product using a mathematical model. The at least one processing property of interest may be a baking property or a rheological property of dough prepared from the ground cereal product. The present invention further provides a portable device for the preparation and spectroscopic analysis of grain samples suitable for performing the prediction method of the invention, and a software product.

Wheat quality describes the suitability or the potential of wheat to be processed and manufactured into a desired end product, e.g., bread, cake or pasta. To determine the quality characteristics of a wheat flour, many different parameters can be analysed, e.g. ash content, falling number, protein content and protein sedimentation properties. Furthermore, various rheological tests on dough prepared from the wheat flour can be carried out, for example using Farinograph, Extensograph, AlveoLab, MixoLab or other devices. An experienced user can draw at least some conclusions about the expected baking behaviour of a flour based on these parameters and tests. However, the parameters mentioned do not always describe the baking properties of a flour in a satisfying manner. The determination of the quality and processing properties of novel flour varieties thus still largely relies on the execution of laborious baking tests. In practice, this is however costly and time-consuming and requires a large amount of flour, which is problematic for mills and breeders in particular (Pojić and Mastilović, 2013).

The relationship between the composition of a wheat flour and its properties, e.g., its baking properties depends on many different factors and is still not fully understood. Often only the protein content of a grain or flour is relied on to estimate its baking properties. For instance, there is a positive correlation between the protein content of a wheat flour and the baking volume. Various correlations are given in the literature, whereby the values are often between $R^2 = 0.49$ and $R^2 = 0.59$ and therefore rather low. In some cases, higher values are obtained, but these were measured on a very small sample set or on single-variety samples from one harvest year. In practice, however, mills very rarely have the opportunity to obtain single-variety wheat. In addition, the properties of flours also change from harvest to harvest, depending, for example, on the weather, irrigation, fertilisation and location.

Because of this, new approaches involve using spectroscopy of wheat kernels and flour in combination with chemometric modeling to improve the prediction of wheat quality characteristics (Nagel-Held et al., 2022; Pojić and Mastilović, 2013). Spectroscopy is a rapid, non-destructive technique that has become increasingly important in science and industrial applications (Lakowicz, 2006). The use of spectroscopic methods, in particular NIR spectroscopy, to predict baking properties from flour spectra was already considered in the 1980s (e.g. Osborne, 1984). Since then, fluorescence and Raman spectroscopy have also been contemplated for the prediction of wheat quality parameters (Ahmad et al., 2016; Nagel-Held et al., 2022; Nawrocka et al., 2016). However, the predictive power of flour spectra is only slightly better than that of the protein content and therefore not high enough to replace baking tests.

Rubenthaler and Pomeranz (1987) obtained better results by correlating different wavelengths. However, according to investigations by Delwiche and Weaver (1994), these results were influenced by the selection of the samples and therefore not very informative for practical use. In the latter publication, flour samples in the wavelength range from 1100 nm to 2498 nm were measured in steps of 2 nm using NIR spectroscopy. The correlation between the measured spectra and the height of a loaf baked from the flour was determined using both principal component analysis and partial least squares regression. However, the conclusion from the experiment was that the applicability of NIR spectroscopy for determining bread volume is low.

In WO 2002/054061 A1, Bengtsson describes a method in which baking properties are predicted by performing multivariate analysis of NIR spectra of flour samples in the wavelength range of 400 - 2500 nm.

Sorvaniemi et al. (1993) describe a method in which Scanning Fourier Transform Near Infrared Reflectance (FT-NIR) spectroscopy is used to predict quality parameters of flour. In particular, moisture and protein content could be determined with a high correlation (coefficient of determination $R^2 = 0.97$ and 0.92 respectively). The baking properties of the flour were however not predicted in this way.

Nagel-Held et al. (2022; 2024) recently analysed the largest sample set to date with almost 1200 flour samples and tried to predict the baking volume of these samples using different spectroscopic methods. However, the predictive ability of their approach was rather poor, achieving correlations $R^2$ of merely 0.61 using NIR spectroscopy of flour, an $R^2$ of 0.59 using Raman spectroscopy and an $R^2$ of 0.57 using fluorescence spectroscopy for the prediction of the specific volume.

In light of the state of the art, there is thus a need for a method which facilitates an easier, more rapid, and, most importantly, a more reliable prediction of the baking properties of a flour than the current methods of the state of the art and without the need for conducting baking tests for each sample. Such a method that can be used to accurately determine the baking properties of a particular flour in a short time would be an extremely valuable tool for both mills and industrial bakers.

The problem is solved by the present invention, in particular by the subject matter of the present claims. The present invention discloses a novel approach that relies on the spectroscopic analysis of flour fractions rather than merely whole flour samples for predicting the processing properties of a flour.

In particular, in a first aspect, the present invention relates to a method for predicting at least one processing property of interest of a ground cereal product, comprising steps of:

a) providing a ground cereal product;
b) separating the ground cereal product into at least two distinct fractions according to at least one parameter;
c) analysing at least one of these fractions by spectroscopy to obtain at least one spectrum;
d) on the basis of the at least one spectrum obtained in step c), predicting the at least one processing property of interest of the ground cereal product provided in step a) using a mathematic model, wherein the mathematic model is obtainable by correlating spectra of fractions corresponding to the at least one fraction analysed in step c) from a plurality of reference ground cereal products that are different from the ground wheat product provided in step a) with the at least one processing property of interest that has been previously determined for each of the plurality of reference ground cereal products.

The ground cereal product which is provided in step a) and for which the at least one processing property of interest is to be determined can be a novel and, thus, a yet unknown or little-known ground cereal product. For example, it may be a flour produced from a new wheat variety or obtained from known wheat varieties grown under intentionally modified cultivation conditions. As the properties of well-known flours can also change from harvest to harvest, depending, e.g., on the weather, irrigation, fertilisation and location during cultivation, the method of the present invention may also be used to predict at least one processing property of a ground cereal product, e.g., from an established wheat variety, e.g., originating from a fresh harvest. The method of the invention can be used to determine possible or optimum uses and/or price of the cereal, e.g., the wheat or ground cereal product.

For the sake of simplicity, the ground cereal product provided in step a) for which the at least one processing property is to be predicted is also referred to herein as "sample flour" or "test flour". However, in the context of the invention, the term "ground cereal product" refers not only to cereal flour, but also extends to cereal semolina or a mixture of flour and semolina. Likewise, the term "ground wheat product" refers not only to wheat flour, but also extends to wheat semolina or a mixture of flour and semolina.

"Flour" refers to a fine powder obtainable by grinding or milling raw plant products such as wheat grains to a particle size of preferably less than 150 μm, e.g. of 50-149 μm. Smaller particles may also be contained. Flour, e.g., wheat flour may either be whole grain, i.e., it may be prepared from the endosperm, germ and bran together, or it may be a refined flour, i.e., it may be prepared only or partly from the starch-rich endosperm. Accordingly, the ground cereal product provided in step a) may be whole grain flour or refined flour, preferably refined flour.

Semolina refers to the more coarse, purified millings of various cereals including wheat, rice or maize. It is however typically obtained from milling hard wheat species, e.g. *T. durum.* The average particle size of semolina and flour may vary considerably depending on the employed milling technique and the type of wheat used. However, the particle size of semolina is larger than that of flour, commonly in the range of more than 250 μm to about 1000 μm, e.g. from about 300 μm to about 750 μm (en.wikipedia.org/wiki/Semolina; Sacchetti et al., 2011). However, ground cereal grain products with particle sizes smaller than 250 μm are often still referred to as semolina. In the context of the invention, the ground cereal product is therefore considered to be semolina if it has a particle size of more than 150 μm, e.g. of 150-1000 μm. During semolina production from wheat, the bran and germ of the wheat are flaked-off, while the starch-rich endosperm is cracked into coarse fragments. These endosperm pieces form the actual semolina when separated from the bran. The semolina can optionally be further ground into finer particles to produce flour.

In the context of the invention, the mean particle size is preferably analysed by sieving using, e.g., a test sieve shaker such as a Ro-Tap® (www.haverparticleanalysis.com/en/sieve-analysis/ro-tapr-test-sieve-shaker/), Vibratory Sieve Shaker AS 200 Control (www.retsch.com/products/sieving/sieve-shakers/as-200-control/function-features/). An air jet sieve such as the Laboratory Air-jet Lab sieve KLS (gkm-net.de/en/laboratory-air-jet-lab-sieves.html) can also be used. Alternatively, particle size may also be determined by laser diffraction or spectrometry (Hareland, 1994).

In a preferred embodiment, the ground cereal product is flour with an average particle size of less than 150 μm, more preferably less than 125 μm, less than 110 μm or less than 100 μm. E.g., at least 50 %, preferably at least 75 %, at least 80 %, at least 85 %, at least 90 %, at least 95 %, at least 96 %, at least 97 %, at least 98 % or at least 99 % of the ground cereal product is provided as flour e.g., as defined herein. Preferably, 100 % of the ground cereal product is flour. Accordingly, typically, less than 50 %, preferably, less than 25 %, less than 20 %, less than 15 %, less than 10 %, less than 5 %, less than 4 %, less than 3 %, less than 2 % or less than 1 % of the ground cereal product are provided as semolina. Preferably, the ground cereal product tested in the method of the present invention does not comprise any semolina.

In some embodiments, the proportion of semolina may however also be higher, e.g., at least 25 %. It may for instance constitute at least 30 %, at least 35 %, at least 40 %, at least 45 % or at least 50 % of the ground cereal product. In some embodiments, the majority of the ground cereal product may be provided as semolina, e.g. at least 60 %, at least 70 %, at least 80 %, at least 90 %, at least 95 %, at last 95 %, at least 96 %, at least 97 %, at least 98 % or at least 99 %. Optionally, the entire ground cereal product is provided as semolina. Typically, the semolina has an average particle size larger than 150 μm, preferably larger than 200 μm or larger than 300 μm, more preferably, larger than 400 μm, larger than 450 μm, or larger than 500 μm.

The term "cereal" refers to a grass cultivated for its edible grain, i.e., the dry fruit (caryopsis) of the cereal plant.

In principle, the cereal can be any type of cereal, e.g., it may be wheat, rye, oat, barley, millet, rice or maize. However, preferably, the cereal is wheat. Accordingly, in a preferred embodiment of the method of the invention, the ground cereal product is a ground wheat product. The wheat may be any type of wheat, e.g., it may be *Triticum aestivum, Triticum durum, Triticum dicoccum, Triticum spelta, Triticum monococcum* or a mixture thereof. However, preferably, the wheat is *Triticum aestivum.*

*Triticum aestivum,* better known as common wheat or bread wheat, constitutes approximately 90 % of the globally produced wheat. *T. aestivum* is occasionally also differentiated into soft wheat and hard wheat. Soft wheat refers to *T. aestivum* with a lower gluten content than hard wheat. *T. aestivum* is a hexaploid wheat species and contains on average about 9-12 %, e.g., 11 % protein (dry weight) (Zilic et al., 2011). Gluten constitutes about 75-85 % of said total grain proteins and stores carbon, nitrogen and sulphur to support seed germination. Gluten is a protein mixture composed of prolamins and glutelines, which, in the context of wheat, are usually referred to as gliadins and glutenins, respectively. Glutenins form protein aggregates stabilized via intermolecular disulfide bonds that become attached to the monomeric gliadins. Collectively, gluten proteins form a matrix with viscoelastic and adhesive properties (Shewry et al., 2002). *T. aestivum* carries rather softer grains than durum wheat that can be conveniently milled into fine flour. As a result, the gluten networks become exposed and can be easily brought together during the mixing and kneading of dough made of bread wheat, thereby creating new protein-protein interactions within the growing gluten network. Accordingly, doughs prepared from *T. aestivum* are usually high in strength and elasticity and are therefore preferred for bread making.

*T. aestivum* can also be used for baked products other than bread, e.g., for cakes, cookies, biscuits, or wafers. For these applications, flour from softer *T. aestivum* variants is preferred. *T. aestivum* of intermediate hardness is preferred for the preparation of flour that can be used in laminated crackers and hard biscuits.

*T. durum* is a tetraploid wheat species, derived from the tetraploid species *Triticum dicoccum,* and the second most cultivated species of wheat after *T. aestivum.* The Latin name Durum translates as hard, as the grains of *T. durum* are known for being highly resistant to milling. *T. durum* is therefore mostly processed into more coarse-grained semolina rather than into fine flour. *T. durum* is described to contain slightly higher levels of gluten compared to *T. aestivum* (Zilic et al., 2011). However, because the gluten- and starch-rich endosperms are often only partly cracked in durum semolina, the gluten is less readily available. Doughs made from durum wheat semolina are therefore less elastic and sticky than those made from bread wheat flour. Accordingly, semolina made from *T. durum* is only rarely used for baking bread and is instead particularly suitable for making pasta and couscous.

*Triticum spelta,* also referred to as spelt or dinkel wheat, is a relict crop, which enjoys growing popularity among more health-oriented consumers. Spelt is a hexaploid wheat and is most commonly used for baking breads, rolls and other pastries, as it shares many properties with *T. aestivum.*

*Triticum dicoccum,* better known as emmer wheat or hulled wheat, is considered to be one of the oldest domesticated crop species. Emmer is a particular stress-resistant wheat and therefore can grow on comparably poor soils. Similar to *T. durum,* emmer possesses relatively hard grains. Emmer wheat is most famously employed in the production of a particular type of Italian bread (pane di farro).

Similar to emmer and spelt, *Triticum monococcum* (einkorn wheat) is an ancient wheat and is characterized by a high protein and fat content. It is commonly consumed in Provence, France, and may be used as an ingredient of bulgur.

In the present invention, the ground wheat product for which the at least one processing property of interest is to be predicted using the method of the present invention preferably is flour from *Triticum aestivum,* because it is by far the most cultivated wheat species in the world and therefore far more available than *T. durum, T. dicoccum, T. spelta* and *T. monococcum.*

The ground wheat product may, however, also be a combination of different wheat species, e.g., it may be a mixture of *T. aestivum* and *T. durum.* Preferably, the ground wheat product comprises more *T. aestivum* than *T. durum.* The percentage of *T. aestivum* in the ground wheat product may be, e.g., 0-100 %, e.g., 90-100 %, 80-90 %, 70-80 %, 60-70 %, 50-60 %, with *T. durum* ad 100 %. % refers to a weight per weight ratio (wt/wt) unless otherwise mentioned. In other embodiments, the ground wheat product may however also comprise more *T. durum* than *T. aestivum,* i.e., the percentage of *T. aestivum* being 40-50 %, 30-20 %, 20-30 %, 10-20 % or 0-10 %, with *T. durum* ad 100 %. It may also comprise only *T. durum* and no *T. aestivum.*

The ground wheat product may however also comprise one or more of *T. dicoccum, T. spelta* and/or *T. monococcum*, optionally, in a mixture with *T. aestivum.* The ground wheat product may therefore also have a *T. dicoccum* content of, e.g., 0-100 %, e.g., 90-100 %, 80-89 %, 70-80 %, 60-70 %, 50-60 %, 40-50 %, 30-40 %, 20-30 %, 10-20 % or 0-10 %, with any other of the herein described wheat species, e.g., *T. aestivum,* ad 100 %. The ground wheat product may therefore also have a *T. spelta* content of, e.g., 0-100 %, e.g., 90-100 %, 80-89 %, 70-80 %, 60-70 %, 50-60 %, 40-50 %, 30-40 %, 20-30 %, 10-20 % or 0-10 %, with any other of the herein described wheat species, e.g., *T. aestivum,* ad 100 %. The ground wheat product may also have a *T. monococcum* content of, e.g., 0-100 %, e.g., 90-100 %, 80-89 %, 70-80 %, 60-70 %, 50-60 %, 40-50 %, 30-40 %, 20-30 %, 10-20 % or 0-10 %, with any other of the herein described wheat species, e.g., *T. aestivum,* ad 100 %.

In the context of the invention, the "processing property" of interest that is to be predicted for a ground cereal product using the method of the present invention refers either to a baking property of the ground cereal product or a rheological property of dough prepared from the ground cereal product.

The term "baking property" is herein understood to mainly relate to any property or characteristic of a baked good, e.g., of bread or of a confectionary good, that is produced from the ground cereal product, i.e., the "test flour". Well-established baking properties of ground cereal products, in particular ground wheat products, that are widely associated by bakers and end-consumers with wheat quality include, e.g., baking volume, bread texture or bread colour.

The term "baking volume" refers to the specific volume of a loaf of bread, a roll or a confectionary product, i.e. the volume yield of a defined quantity of flour, e.g. 100 g, that has been baked from a dough prepared with the ground cereal product, e.g., the ground wheat product. In the context of the invention, the term is typically used to refer to the volume of a loaf of bread, made from a given amount of flour, as specified in the recipe that is being used. The baking volume, which is sometimes also referred to as "loaf volume" when describing the volume of baked bread, depends, inter alia, on the protein content of the grain from which the ground cereal product is made. The skilled person is aware of various standardized methods for determining baking volume. Some methods, such as the method according to Neumann/Doose or standard method AACC 10-05.01 of the Cereals & Grains Association, rely on the displacement principle (such as rapeseed displacement) to measure baking volume. Alternatively, baking volume may be determined using volume scanners that rely on laser distance sensors to scan the upper surface of the baked goods. Since baking volume is such a well-established and readily determinable quality parameter of cereals, in particular of wheat, the processing property of interest of the ground cereal product to be predicted by the method according to the invention is preferably baking volume.

Bread texture can be expressed, e.g., as crumb firmness, stiffness or relative elasticity of a baked bread and is typically evaluated using special texture analyser device distributed, e.g., by Stable Micro Systems. Standardized methods for assessing bread texture are well-known to the skilled person and include, e.g., the methods according to AACC 74-09.01 or AACC 74-10.02 of the Cereals & Grains Association.

Bread colour can refer to both the colour of the crust and that of the crumb of a baked bread and can be precisely determined using, e.g., a spectrophotometer.

However, in the context of the present disclosure, the term "baking property" not only relates to properties of the final baked goods produced from the ground cereal product but may also extend to characteristics of the ground cereal product itself that are known to influence the baking quality.

Examples of such characteristics include the water absorption ability of the ground cereal product or the flour falling number.

Water absorption refers to the amount of water that can be taken up by the particles of a ground cereal product, e.g., the ground wheat product. Water absorption directly impacts, e.g., baking volume, fracture stress of the bread crumb or the shelf life of the final baked good. It can be conveniently measured using specialized laboratory equipment such as, e.g., a Farinograph using standardized methods known to the skilled person, e.g., ICC-Standard 115/1 or AACC 44-01.01.

The falling number, also known as Hagberg number or Hagberg-Perten number, is a standardized (ICC 107/1, ISO 3093-2004, AACC 56-81B) way for determining the intrinsic alpha-amylase activity of a grain or its flour. Alpha-amylase is low in sound grain but increases with premature sprouting (i.e., undesirable germination) in wheat due to damp or rainy weather conditions during the final stage of maturation of the crop. The falling number method reflects the viscosity of the starch gel formed in a flour suspension in a test tube heated in a boiling water bath. Intrinsic alpha-amylase partially hydrolyses the gel before being inactivated by heating, thereby reducing the viscosity. The falling number is the time (in seconds) taken for a standardised plunger to pass through the probe plus 60 seconds. Wheat flours with medium falling numbers (230-280 seconds) produce a normal, elastic, well-pored crumb in the final bread and have sufficient leavening power. Flours with low falling numbers (<220 s) produce moist, sticky doughs with low mixing tolerance and bread with an inelastic, moist crumb. Due to the lack of stability, the volume yield may be low and product shape may be compromised. Wheat flours with a high falling number (>300 s), on the other hand, produce a dry crumb and a low baking volume due to the lack of fermentable sugars.

In some embodiments, the term "baking property" may also encompass the cooking properties of products made from the ground cereal product. Thus, when the term "baking property" is used in the context of the present invention, it may also refer to the properties of cooked dough products, e.g., the firmness or stickiness of cooked pasta that has been prepared, e.g., from durum wheat. The firmness of cooked pasta can be assessed using, e.g., standard methods AACC 66-50.01, AACC 66-51.01 or AACC 66-52.01 of the Cereals & Grains Association.

In some embodiments, the at least one "processing property" of interest of a ground cereal product that can be predicted using the method of the invention may also be a rheological property of dough prepared from the ground cereal product. Rheology deals with the deformation and flow behaviour of matter. Exemplary rheological properties of dough that can be predicted using the method according to the invention therefore include dough extensibility, dough stretch resistance, dough kneading or mixing tolerance, dough stability and gelatinisation behaviour. Based on the rheological properties of the dough, important predictions can be made about the behaviour of the dough during baking, e.g. whether a flour is more suitable for making bread, rolls or biscuits.

The elongation properties (e.g., dough extensibility or stretch resistance) of a wheat dough can be measured using specialized laboratory equipment, e.g., an Extensograph according to standard method ICC 114/1 or an alveograph according to standard method ICC 121/2. When using an Extensograph, a dough produced under standardised conditions is first formed into a dough strand and, after a short resting phase, is stretched after defined time points until it tears. The force required for this is recorded in the form of a curve, a so-called Extensogram that can be interpreted by the skilled person.

Kneading/Mixing tolerance of a dough prepared from the ground cereal product refers to the resistance of the dough to breakdown during continued kneading or mixing. The mixing tolerance of dough prepared from, e.g., a ground wheat product can be determined with, e.g., a Mixograph using standardized methods known to the skilled person, e.g., AACC 54-40.02 or a Farinograph using standard method AACC 54-21.02.

Stability of a dough prepared from the ground grain product is an indication of dough strength as it is a measurement of how long the dough maintains maximum consistency. Dough stability is expressed in minutes and can be determined for dough prepared from, e.g., ground wheat products with a Farinograph using standard method AACC 54-21.02.

Gelatinisation describes the property of flours to stick together in the presence of water and heat due to their starch content and the activity of alpha-amylases. Gelatinisation properties of wheat flours can be determined with, e.g., an Amylograph using standard method ICC 126/1. In this method, a suspension of flour and water is continuously heated under stirring, causing the starch in the flour to gelatinise and the viscosity of the suspension to increase. This gelatinisation is recorded by the Amylograph in the form of a curve, a so-called amylogram.

In some embodiments, the method of the present invention is used to predict more than one processing property of the ground cereal product, e.g., two, three, four, five, six, seven, eight, nine, or ten processing properties. For example, baking volume, bread texture and bread colour can be predicted. In some embodiments, all of the herein disclosed processing properties of the ground cereal product are predicted using the method of the invention.

To predict the at least one processing property of interest, the method according to the invention requires the ground cereal product, i.e. the "test flour/semolina", to be separated into at least two different fractions in step b), a process also referred to herein as fractionation. The fractionation is performed according to at least one parameter, i.e., it is not a random fractionation.

Fractionation may be achieved according to different parameters, and by different means. In a preferred embodiment, the ground cereal product is fractionated based on particle size, i.e., the ground cereal product is separated into at least two fractions, wherein the individual flour/semolina particles in the first fraction are smaller or larger than the particles in the second fraction. Alternatively, fractionation during step b) of the method of the invention may be based on the density of the different particles of the ground cereal product, i.e., the particles in a first fraction have a lower or higher density than the individual particles in the second fraction. Fractionation in step b) may also be achieved based on particle shape or composition, e.g., based on protein or starch content. The fractionation may also depend on a combination of parameters, e.g., a combination of size and density.

Previous studies have shown that protein enrichment and the enrichment of polyphenolic compounds in various fractions is possible by separating flour particles according to particle size and/or density (Jensen et al., 1982; Jones et al., 1959; Youssef and Abo-Dief, 2022). Thus, without being bound by theory, the inventors believe that the enrichment or depletion of certain compounds by fractionation reduces the strong superimposition of spectral signals that normally take place when unfractionated flour or semolina samples are spectroscopically analysed, which might in turn improve the reliability of the predictions on, e.g., wheat quality.

The skilled person will be aware of various different methods suitable for separating a ground cereal product into distinct fractions. For instance, fractionation can be achieved via a method selected from the group comprising sieving, air classification or tribo-electrostatic separation.

In a preferred embodiment, fractionation during step b) of the method of the invention is achieved via sieving, i.e., by passing the ground cereal product through one or more sieves, each comprising a plurality of holes of the same or substantially same size that form the sieve mesh. The size, i.e., the diameter of the holes in a sieve mesh determines the "mesh size" of the sieve: The larger the diameter of the holes, the greater the mesh size of the sieve.

In some embodiments of the invention, the fractionation via sieving relies on the use of only a single sieve of a given mesh size, which means that the ground cereal product is separated into two different fractions. The first fraction consists of grains or particles with a particle size smaller than the sieve's mesh size which will therefore pass through the holes of the sieve. This fraction is commonly also referred to as sieve passage. The second fraction consists of particles with a size larger than the sieve's mesh size, which is why it is intercepted by the sieve where it forms the so-called sieve overflow.

However, preferably, the ground cereal product is passed during step b) through a plurality of sieves of different mesh sizes, e.g. two, three, four, five, six, seven, eight, nine, ten or more sieves of different mesh size to ultimately obtain more than two distinct fractions, e.g., three, four, five, six, seven, eight, nine, ten or more fractions. In such embodiments, the ground cereal product is first passed through the sieve with the largest mesh size. The flour particles retained by this first sieve form the first fraction, whereas those particles that are small enough to pass through the first sieve are then sieved with a second sieve having a mesh size that is smaller than the mesh size of the first sieve. The flour particles small enough to pass the first sieve but too large to pass the second sieve form the second fraction. At the same time, those flour particles passing the first and the second sieve form the third fraction. The third fraction can optionally be subjected to further rounds of sieving using sieves with increasingly smaller mesh sizes until the desired number of fractions has been obtained.

Sieving is typically achieved by vibrating the sieves, i.e., the sieves are mechanically moved by, e.g., shaking, rattling or tapping to promote the passing of the ground cereal product through the sieves and therefore the separation of the ground cereal product by grain size.

In a particular preferred embodiment, the fractionation step b) of the method of the invention may be achieved using air-jet sieving. Especially when working with finer flours having smaller particle sizes, there is an increased risk that individual grains will agglomerate and clog the sieve holes, which interferes with the fractionation process. Air-jet sieving does not rely on mechanical motions but uses streams of air to move particles through a sieve mesh. To do so, a strong jet of air is blown through the sieve to disperse the flour particles and distribute them over the complete sieve surface. Particles with sizes smaller than the sieve's mesh size are then sucked through the mesh by a vacuum. Air-jet sieving can be performed using special equipment well known to the skilled person, e.g., the air jet sieving machine AS 200 JET by Retsch® or the Laboratory Air-jet Lab sieve KLS by GKM Siebtechnik.

As alternative to sieving, the ground cereal product may also be fractionated, e.g., by air classification or tribo-electrostatic separation.

Air classification refers to a pneumatic fractionation method for fractionating particles based on their size and density. The ground cereal product that is to be fractionated is fed into a chamber using air currents, which simultaneously fluidize the ground cereal product and induces centrifugal and gravitational forces, i.e., the air drag supplies an upward force which counteracts the force of gravity and lifts the ground cereal product into the air. Due to the dependence of air drag on object size, shape and density, the flour/semolina particles in the moving air column are sorted vertically and can be separated in this manner (https://en.wikipedia.org/wiki/Air_classifier).

During tribo-electrostatic separation, the particles of the ground cereal product are first electrically charged by suspending the particles in a gas stream in the form of a diluted phase, where constant collisions between particles and between particles and the walls of the process equipment build up electrical charge on the surface. This process is also known as triboelectric charging. Afterwards, an electric field is applied to the thus charged particles of the ground cereal product. The particles will follow a different path through this electric field according to their charge magnitude and polarity, while uncharged particles will have their trajectories unchanged. Thus, the separation of the ground cereal product into distinct fractions is not based on particle size or density - as in conventional dry separation processes - but on the different triboelectric chargeability of the particles' surfaces.

In further embodiments of the invention, the ground cereal product may also be separated into fractions based on its ingredients, e.g., based on its protein composition. A person skilled in the art is aware of various methods for achieving this. For example, proteins present in a ground wheat product can be fractionated according to their solubility using Osborne fractionation. Albumins contained in wheat (e.g., leucosin) can be extracted with water, globulins (e.g., edestin) with saline solution and prolamins (e.g., gliadin) by using 70% ethanol. Glutelins such as gluten-in remain in the residue as they cannot be extracted. However, Osborne fractionation is associated with the disadvantage that residues of the solvents used can impair the subsequent spectroscopic analysis of the different fractions during step c) of the method of the invention, which is why this form of fractionation is less preferred in the context of the invention.

In other embodiments, fractionation during step b) is carried out after the ground cereal product has been processed into a dough. For example, gluten and starch fractions can be extracted from a dough prepared from the ground cereal product, e.g., a wheat flour or semolina, by washing. In brief, a dough is first prepared by mixing the ground cereal product with a saline solution. The starch is then washed out of this dough with saline as a first fraction until a yellowish dough residue remains, which contains the gluten fraction. In some embodiments, the ground cereal product is a ground wheat product, and the dough can be produced from the ground wheat product in a Farinograph in accordance with ICC standard 115, which is then washed, freeze-dried and ground in accordance with ICC 155. Washing out of gluten from a dough may also be achieved using, e.g., a Perten Glutomatic 2202 system from PerkinElmer.

Preferably, fractionation is carried out directly on the ground cereal product without previous extraction or processing, e.g., into dough.

As already mentioned herein, the ground cereal product is preferably separated into more than two fractions, e.g., it is separated into three, four, five, six, seven, eight, nine, ten or even more distinct fraction. For instance, in some embodiments and as described in the Examples below, the ground cereal product can be separated in step b) into five distinct fractions based on particle size, e.g., via sieving such as air-jet sieving, wherein the first fraction consists of particles with a diameter of less than 32 μm, the second fraction consists of particles with a diameter of 32-50 μm, the third fraction consists of particles with a diameter of 50-75 μm, the fourth fractions consists of particles with a diameter of 75-100 μm and the fifth fraction consists of particles having a diameter of more than 100 μm.

Some or all of the specific fractions described above are preferred when the ground cereal product to be fractionated is a flour with a particle size of less than 150 μm, as defined herein. However, it is to be understood that these specific fractions should only be considered as examples. The skilled person will be able to divide the ground cereal product into at least two fractions consisting of particles of sizes other than those indicated above, which can be achieved simply by changing the mesh size of the sieves used for fractionation. In particular, if the ground cereal product to be fractionated is a coarser-grained semolina sample consisting of particles larger than 150 μm rather than fine cereal flour, the skilled person will select the fractions accordingly. For example, when fractionating cereal semolina, a first fraction can e.g., consist of particles with a size of < 200 μm, a second fraction of particles with a size of 200-250 μm, a third fraction of particles with a size of 250-300 μm, a fourth fraction of particles with a size of 300-350 μm, a fifth fraction of particles with a size of 350-400, a sixth fraction of particles with a size of 400-450 μm, a seventh fraction of particles with a size of 450-500 μm and an eighth fraction of particles with a size of >500 μm.

Irrespective of the specific fractionation method selected and the number of different fractions obtained in step b) of the method according to the invention, at least one of these fractions is analysed spectroscopically in the following step c) to obtain at least one spectrum.

In some embodiments, it may be sufficient to analyse only a single fraction spectroscopically to make a reliable prediction regarding any of the herein described processing properties of the ground cereal product. Various tests carried out by the inventors have shown, e.g., that a fraction consisting of flour particles with a size of 50-75 μm, which can be obtained by sieving of a wheat flour, is particularly enriched in protein compared to the unfractionated flour sample. The analysis of this specific fraction could therefore already be sufficient to increase reliability of predictions regarding the rheological properties of doughs made from the ground cereal product or its baking volume, if said ground cereal product is a flour comprising or consisting of particles with an average diameter of less than 150 μm, as defined herein.

In a preferred embodiment, however, at least two fractions are analysed in step c). The subsequent analysis comprises analysis of the at least two spectra obtained to achieve more reliable predictions. Thus, depending on how many different fractions the ground cereal product was separated into during step b), at least two, e.g., at least three, at least four or at least five fractions can be analysed spectroscopically during step c) of the method according to the invention. Accordingly, the corresponding number of spectra is obtained, and typically, subsequently analysed. In some embodiments of the invention, all fractions of the ground cereal product obtained in step b) are analysed in step c).

Analysis of the at least one fraction of the ground cereal product during step c) of the method of the invention is achieved via spectroscopy. Spectroscopy refers to a group of physical methods that break down and analyse radiation according to a specific property such as wavelength, energy, mass, etc. The resulting intensity distribution is called a spectrum.

During spectroscopy, electromagnetic radiation within wavelength ranges of, e.g., 250 to 2500 nm, such as 250 to 700 nm, 350 to 1000 nm, 500 to 1500 nm, 1500 to 2000 nm or 2000 to 2500 nm is directed at the sample, i.e., the at least one fraction of the ground cereal product. This process is also referred to as excitation. The radiation will interact differently with the different molecules present the sample.

The skilled person is readily able to select and, if necessary, optimize suitable wavelengths for the spectroscopic analysis of the at least one fraction depending on its composition and/or properties. Preferably, the spectrum of the at least one fraction of the ground cereal product is measured not only at a single wavelength but at a plurality of wavelengths. Accordingly, for each fraction that is spectroscopically analysed in step c), preferably a plurality of spectra at different wavelengths is obtained. For example, the spectrum for the at least one fraction can be measured in a wavelength range of between 250 and 2500 nm in steps of at least 10, at least 20, at least 30, at least 40, at least 50, at least 60, at least 70, at least 80, at least 90 or at least 100 nm. Preferably, the spectrum for the at least one fraction is measured at as many wavelengths as possible, e.g. at 5-100 wavelengths, i.e. at least five, at least ten, at least 15, at least 20, at least 25, at least 50, at least 75 or even 100 wavelengths. A scan at different wavelengths, e.g., over the ranges mentioned above, can also be carried out.

The skilled person will be able to choose from various spectroscopic methods that are suitable for analysing the at least one fraction of the ground cereal product. For instance, NIR-spectroscopy, fluorescence spectroscopy or Raman spectroscopy may be used in the context of the present invention.

In case of fluorescence spectroscopy, so-called fluorophores, i.e., photoreactive chemical compounds present in the sample, absorb the excitation light energy of a certain wavelength and emit this light at a longer wavelength, which can then be measured. Exemplary fluorophores that are naturally present in ground cereal products include but are not limited to, e.g., the amino acid tryptophan, phenolic acids (especially ferulic acid), different oxidation products of lipids, vitamins and phenolic acids, carotenoids, riboflavin, flavin adenine dinucleotide (FAD) and flavin mononucleotide (FMN).

As the name implies, near-infrared (NIR) spectroscopy is a spectroscopic method that uses the near-infrared region of the electromagnetic spectrum (from 780 nm to 2500 nm). The incident electromagnetic radiation probes molecular vibrations, i.e. oscillating changes in bond lengths (stretching vibrations) and angles between these bonds (deformation vibrations).

Similar to NIR spectroscopy, Raman spectroscopy belongs to the group of vibrational spectroscopies in which vibrations in various molecular bonds of the sample are indirectly excited by the light through Raman scattering.

Independent of the precise method chosen, spectroscopy produces a signal as a function of wavelength that contains information about the composition of a sample, i.e., the composition of a given fraction of the ground cereal product.

Optionally, in addition to the spectroscopic analysis of the at least one fraction of the ground cereal product in step c), the method according to the invention may also comprise the spectroscopic analysis of the whole, i.e., the non-fractionated ground cereal product. When being combined with the spectroscopic analysis of one or more fractions of the ground cereal product, the spectroscopic analysis of the unfractionated ground cereal product can further increase the predictive power of the method according to the invention. Spectroscopic analysis of the unfractionated ground cereal product can be carried out by the same methods and under the same conditions as the analysis of the individual fractions described herein.

The processing property of interest, e.g., a baking property such as baking volume of a ground cereal product can only be predicted using the method of the invention if the results of the spectroscopic measurements of the at least one fraction obtained in step c) are evaluated in light of empirical data derived from the spectroscopic analysis of corresponding fractions of other ground cereal products for which the processing property of interest is already known. In other words, the method of the present invention thus relies on mathematical models obtained via chemometrics to predict the processing properties of interest based on measured properties of known chemical systems.

Therefore, during step d) of the method of the invention, a mathematical model is used to predict the at least one processing property of interest of the ground cereal product, i.e., the "test flour" or "test product" based on the at least one spectrum obtained in step c). This mathematical model is obtainable by correlating the spectra of fractions corresponding to the at least one fraction analysed in step c) from a plurality of reference ground cereal products, e.g., a plurality of reference wheat products that are different from the ground cereal product, e.g., the ground wheat product provided in step a) with the at least one processing property of interest that has been previously determined for each of the plurality of reference ground cereal products. In this context, "fractions corresponding to" means that the fractions have been obtained from ground cereal products separated into at least two fractions according to the same parameter as in the analysis carried out for the test product. For example, if for obtaining the mathematical model, the ground cereal product separated according to size, e.g., from a fraction of 50-100 $\mu$m is analysed to obtain a spectrum, the same size fraction is used for the analysis of the method of the invention. If it is not possible to use exactly the same fractionation parameters, fractions with the most similar size possible are compared to obtain the best possible results, e.g., an at least overlapping size range. The spectra compared by the mathematical model are also obtained using the same spectroscopic method, and, e.g., using the same or substantially the same wavelengths for analysis.

In particular, the mathematical model used for predicting the at least one processing property of interest of the ground cereal product is obtainable by the steps of:

i. providing a plurality of reference ground cereal products (also referred to herein as "reference samples") that are different from the ground cereal product, i.e., the "test flour" for which the at least one processing property is to be predicted;

ii. determine for each of the plurality of reference ground cereal products the at least one processing property of interest using standardized tests;

iii. separating each of the plurality of reference ground cereal products into at least two distinct fractions that correspond to the at least two fractions obtained in step b) of the method of the invention;

iv. analysing at least one of the fractions of each of the plurality of reference ground cereal products by spectroscopy to obtain at least one spectrum, wherein optionally, at least two fractions are analysed in step iv);

v. generating the mathematical model by correlating, for each of the plurality of reference ground cereal products, the results of the spectroscopic analysis obtained in step iv with the at least one processing property determined in step ii.

It is to be understood that step ii may also be performed after step iii and/or iv, or in parallel to steps iii and/or iv. but prior to step v.

In general, a mathematical model is defined as an abstract description of a concrete system using mathematical concepts and language. It specifies a mathematical relationship between one or more random variables and other non-random variables. The mathematical model used in the method of the invention is a probabilistic model, i.e., a statistical assumption (or set of statistical assumptions) that allows for the calculation of the probability that a given sample, i.e., a ground cereal product, exhibits a certain processing property. The mathematical model can be a mathematical model based on pattern recognition and/or machine learning. It can also comprise use of an artificial intelligence.

The plurality of reference ground cereal products used for generating (training) the mathematical model preferably comprises more than 2 different ground cereal products, e.g., more than 5, more than 10, more than 20, more than 30, more than 40, or more than 50 different ground cereal products, preferably, the plurality of reference ground cereal products comprises more than 75, more than 100, more than 200, more than 500, more than 750, or even more than 1000 different ground wheat products. In general, the more different reference ground cereal products are analysed for developing the mathematical model, the more accurate and therefore reliable the prediction of the at least one processing property of interest of the "test flour" will be.

The individual "reference" ground cereal products used to generate the mathematical model should be different from each other, but preferably all from the same type of cereal, i.e., all reference ground cereal products used for generating the mathematical model are, e.g., ground wheat products. Thus, the different reference ground cereal products can be ground wheat products that preferably cover a broad spectrum of different flour/semolina types and variations from a wide range of wheat species and subspecies from various geographical origins. For example, numerous subspecies of *T. aestivum* are cultivated around the globe, including *T. aestivum* L. subsp. *aestivum*, *T. aestivum* subsp. *compactum, T. aestivum* subsp. *hadropyrum*, *T. aestivum* subsp. *macha*, *T. aestivum* subsp. *sphaerococcum* or *T. aestivum* subsp. *vavilovii*. Thus, in some preferred embodiments, the mathematical model is developed based on the analysis of several or all of these *T. aestivum* subspecies.

The mathematical model used for predicting a processing property of interest for any given ground cereal product, i.e., the "test flour", relies on data previously obtained from the plurality of reference ground cereal products. Thus, the choice of cereal used to create the mathematical model determines the type of ground cereal product for which the at least one processing property is to be predicted by the method of the present invention. If, e.g., the plurality of reference ground cereal products is a plurality of different ground wheat products, the "test flour" for which the at least one processing property is to be determined must be a ground wheat product as well. The same principle applies when the reference ground cereal products are, e.g., ground barley products. Accordingly, it is understood that while the plurality of reference ground cereal products used for generating the mathematical model should be different from the ground cereal product, i.e. the "test flour" for which the at least one processing property of interest is to be predicted, the reference ground cereal products and the "test flour" must nevertheless be of the same cereal type, e.g., the reference ground cereal products and the "test flour" are all ground wheat products.

Moreover, when generating the mathematical model, for each one of the plurality of reference ground cereal products, the same at least one processing property of interest has to be determined in step ii. The at least one processing property can be any of the baking or rheological properties described herein, e.g., it can be baking volume. A person skilled in the art will have no problem to, e.g., quantitatively determine the manifestation of a processing property for each reference ground cereal product, e.g. by assigning it a value on a numerical scale or by assigning it into different categories. Preferably, more than one, e.g., at least two, at least three, at least four, at least five, at least six, at least seven, at least eight, at least nine, at least ten or all of the different processing properties described herein are determined for each of the plurality of reference ground cereal products. In any case, the at least one processing property that is to be later predicted for the ground cereal product, i.e., the "test flour" using the herein disclosed method of the invention must be among the processing properties that have been determined for the plurality of reference ground cereal products. Thus, if only one processing property, e.g., baking volume, is determined for the plurality of reference ground cereal products to develop the mathematical model, said mathematical model can only be used to predict that particular one processing property later-on using the method of the invention. Likewise, if more than one of the herein disclosed processing properties, e.g., at least two, at least three, at least four, at least five or all of the herein described processing properties have been determined beforehand for each of the reference ground cereal products when developing the mathematical model, said mathematical model can be adapted and used in the method of the invention to predict all of these processing properties for the ground cereal product, i.e., the "test flour" provided in step a) of the method of the invention.

The at least one processing property should be determined for each of the plurality of reference ground cereal products using standardized tests. Standardized tests are highly controlled tests in which the samples, i.e., the reference ground cereal products, are subjected to the exact same process steps under identical conditions, wherein the test results are analysed using the same criteria.

For example, for standardised baking tests, a dough must first be produced from each of the plurality of reference ground cereal products, whereby all other ingredients used for preparing the dough and the ratio of the individual ingredients to each other must be identical in all tests. The working conditions during dough production, such as temperature, duration and speed of kneading as well as the conditions during the subsequent baking process (temperature, length of baking, humidity, etc.), must also be the same for all samples that are tested and therefore strictly controlled. Otherwise, the comparability of the different samples and the reproducibility of the test results cannot be guaranteed.

In principle, the skilled person is free to choose the exact parameters and criteria for carrying out these standardised tests, as long as they remain the same for all samples.

However, the skilled person can also make use of a variety of devices and standard procedures that facilitate the performance of standardised tests and are internationally recognised. For example, the standardised preparation of a bread dough from *T. aestivum* flour can be carried out according to standard ICC 115 in a Farinograph.

The plurality of reference ground cereal products must be separated into at least two different fractions in step iii using one of the fractionation methods disclosed herein, e.g. sieving, preferably air jet sieving. The exact methods and conditions chosen for the fractionation of the plurality of reference ground cereal products for the establishment of the mathematical model determine the conditions and method to be used later-on for the fractionation of the ground cereal product, i.e. the "test flour" for which the at least one processing property is to be predicted by the method according to the invention. In other words, if the mathematical model has been established on the basis of the analysis of concrete fractions obtained, for e.g., by sieving the reference ground cereal products, the model can be used later only if the "test flour" for which the at least one processing property of interest is to be predicted has been fractionated in the same way, or at least if the fraction, e.g., the size fraction, analysed for the test flour has also been analysed for the reference products.

Likewise, the spectroscopic method and the exact parameters chosen for the analysis of the at least one fraction for each of the plurality of reference ground cereal products used to develop the mathematical model determine how the at least one fraction of the "test flour" is to be analysed in the prediction method according to the invention. Thus, if the mathematical model is based on data obtained, e.g., by NIR spectroscopy of the fractions from the plurality of reference ground cereal products at specific wavelengths, the model can only be used to predict the at least one processing property of a ground cereal product, i.e., the "test flour", if the corresponding fractions of the latter have been analysed in the same way.

The spectra obtained for the different fractions and the data on the processing properties of each of the plurality of the reference ground cereal products (reference samples) serve as training or calibration datasets for building the mathematical model suitable for predicting the at least one processing property of an unknown "test flour". Optionally, in addition to the spectra obtained for the at least one fraction of the reference ground cereal products, also the spectra of the unfractionated reference ground cereal product may be further considered for building the mathematical model. Accordingly, in some embodiments, it may be advantageous to not only spectroscopically analyse the at least one fraction for each of the plurality of reference ground cereal products as described herein but to also spectroscopically analyse the whole, i.e., the non-fractionated reference ground cereal products. When being combined with the spectroscopic analysis of one or more fractions of the ground cereal product, the spectroscopic analysis of the unfractionated whole reference ground cereal products can, in some embodiments, further increase the predictive power of the mathematical model. Spectroscopic analysis of the whole, unfractionated reference ground cereal products can be carried out by the same methods and under the same conditions as the analysis of the individual fractions described herein.

For building the mathematical model, the data of the at least one processing property determined for each of the plurality of reference ground cereal products serve as reference representing the property of interest that is to be predicted later-on using the method of the invention. The multi-wavelength spectral responses obtained for the at least one fraction of each of the plurality of reference ground cereal products in turn represent a measured attribute that corresponds to said property of interest. The skilled person is aware of various multivariate calibration techniques that can be used to construct a mathematical model that relates the multivariate response (i.e., the measured spectra) to the reference (i.e., the processing property determined for each reference ground cereal product), so that the model can be used to efficiently predict the processing property of new samples.

For instance, in principal component regression (PCR), a principal component analysis (PCA) is combined with a multilinear regression (MLR). In PCA, a data set transformed into a number of principal components, each of which describes a part of the variation in the data set. These principal components are then used to create a regression model. Partial least squares regression (PLSR) is based on the same principle, but the principal components are calculated using a different method to PCA, which takes into account not only the variance of the dependent variable, but also the variance of the independent variable.

The inventors have found that the prediction of processing properties, such as baking volume, can be particularly accurate if, regardless of the spectroscopic method used, the spectra of several fractions are taken into account and are correlated with at least one processing property of the reference ground cereal products when creating the mathematical model. Accordingly, it is preferred that the results of the spectroscopic analysis of more than one fraction, e.g. of at least 2, at least 3, at least 4, at least 5 or all fractions of the plurality of reference ground cereal products are correlated with the at least one processing property when developing the mathematical model. The choice and number of the particular fractions of the reference ground cereal products used for building the mathematical model as well as the decision to also consider whole, non-fractionated flour/semolina again determines which exact fractions should be spectroscopically analysed later-on in the method according to the invention to predict the at least one processing property of the "test flour".

Once a mathematical model for the prediction of a processing property as described herein has been established, the model is preferably further calibrated (validated), e.g. via cross-validation, preferably via "leave-one-out cross-validation (LOOCV), a method well known to the skilled person. During LOOCV, the same reference ground cereal products (i.e., the same reference samples) that were used to create the training data for building the mathematic model are re-tested, wherein always one reference sample at a time is excluded from the calibration dataset while the calibration is performed with the remaining reference samples. The reference sample that has been excluded is then predicted using the mathematic model. The procedure is repeated until all reference samples in the data set have been excluded once. The predictions of the excluded reference samples are used to estimate the prediction quality of the mathematical model at the end of the procedure.

The present inventors further found that if the processing property that is to be predicted for a ground cereal product is a baking property such as baking volume as defined herein, the accuracy and reliability of the method of the invention can be further improved when in addition to the spectroscopic analysis of the at least one, preferably two or more fractions of the "test flour", i.e., the ground cereal product provided in step a) of the method of the invention, at least one rheological property of said "test flour" is measured.

Therefore, in a further embodiment of the present invention, the herein disclosed method is a method for predicting at least one of the herein disclosed baking properties of interest of the ground cereal product, wherein the method in addition to steps a)-e) described above further comprises the steps of:

I. measuring at least one rheological property of dough prepared from the ground cereal product provided in step a) of the method of the invention;
II. predicting the at least one baking property of interest of the ground cereal product provided in step a) of the method of the invention using a mathematical model, wherein the mathematical model is further based on the correlation of the at least one rheological property of dough produced from each of the plurality of reference ground cereal products with the at least one baking property of interest that has been previously determined for each of the plurality of reference ground cereal products.

Thus, in this embodiment, the method for obtaining the mathematic model on the basis of which the at least one baking property is to be determined comprises, in addition to steps i.-vi. described above, the steps of measuring at least one, e.g., one, two, three or four, rheological properties for dough prepared from each of the plurality of reference ground cereal products using standard tests and correlating, for each of the plurality of reference ground cereal products, the results of these measurements with the at least one baking property of interest determined in step ii, as disclosed herein.

The at least one rheological property can be any of the herein disclosed rheological properties, e.g., dough extensibility, dough stretch resistance, and dough kneading tolerance. Again, it is understood that the at least one rheological property determined for dough prepared from each of the reference ground cereal products to build the mathematical model determines the rheological property that is to be measured for the "test sample" for which the at least one baking property is to be determined. Moreover, the preparation of the dough and the measurement of the rheological property must be standardized, i.e., they must take place in the same way for all reference ground cereal products as well as the "sample flour". Again, it is preferred that for building the mathematical model, more than one rheological property for dough prepared from each of the reference ground cereal products is determined.

However, as determination of rheological properties for test samples is laborious, it is advantageous that this is not required in the method of the invention.

Preferably, the reliability and accuracy of the mathematical model used for predicting the at least one processing property of a ground cereal product using the method of the present invention is continuously calibrated and thus improved by including new test datasets on further reference ground cereal products, i.e., new flour varieties or by determining, e.g., additional processing properties for each reference ground cereal product and correlating these processing properties with the spectral information and, optionally, rheological data for each reference ground cereal product. For instance, by daily measuring spectroscopic and possibly rheological data of flours over several years in, e.g., bakeries or mills and determining the respective processing properties of the flours by baking tests as described herein, data for different flour samples with different baking properties can be continuously included in the calibration of the mathematical model.

In summary, the present invention describes the development of a mathematical model obtained by correlating processing properties of various reference ground cereal products such as wheat flour or semolina with spectroscopic data collected for fractions of each of these reference ground cereal products and, optionally, rheological data of dough prepared from each of these ground cereal products. The mathematical model is then used for predicting the processing properties of interest of any given ground cereal product as described herein. The disclosed methods are characterized by an unexpectedly high predictive power.

In another aspect, the present invention further provides a device for the preparation and spectroscopic analysis of grain samples, comprising:

- a feed hopper for receiving a grain sample;

- a mill, wherein the feed hopper is configured to transfer the grain sample into the mill, wherein the mill is configured to grind the grain sample,

- a fractionation unit, which is configured to receive the ground grain sample from the mill and to separate the ground grain sample into at least two different fractions,

- a fraction collector, which is configured to receive and store at least one of the fractions of the ground grain sample separately from other fractions from the fractionation unit, and

- a spectrometer, which is joined to the fraction collector and is configured to receive and analyse the at least one fraction separately from other fractions.

The device is preferably portable, i.e., of such a size and weight that it can be comfortably carried and operated by a user, e.g., with one hand. For example, the device can have a maximal dimension of at most 1 m, at most 50 cm or at most 30 cm. For example, it can have a length of 15-30 cm, a width of 5-10 cm. The weight can be less than 5000 g, preferably, less than 1000 g, e.g., less than 750 g or less than 500 g. The device is therefore preferably a hand-held device. The device can also be larger and heavier than a typical hand-held device, as long as it is portable for an average human user, e.g., inside a suitcase. The device may be formed in one piece, i.e., the individual components are connected to each other in such a way that they cannot be separated from each other without damaging the device. The different components of the device may also be at least partially contained within a one-piece housing. In alternative embodiments, some or all of the components of the device are separably connected to each other using suitable connecting means, e.g., screws or clamps. Therefore, in some embodiments, it may be possible to store and transport the device in a disassembled state and only assemble it, e.g., prior to its use.

As the device is portable, it can be easily transported and used at any location, e.g. directly in a field where the grain that is to be analysed using the device according to the invention is grown, or in a mill. To ensure the power supply of the device, the device preferably comprises one or more batteries. The batteries may be removable by the user or permanently installed with the device. In the latter case, the battery is rechargeable, and the device further comprises a connection socket for a cable to charge the battery. In addition, the device may further comprise one or more solar panels on its surface that converts sunlight into electricity for powering the device.

As a first component, the device comprises a feed hopper or funnel on one of its surfaces that can be filled with grain by the user. The feed hopper should comprise an opening that is sufficiently large to receive the grain. The diameter of this opening can be about 5-20 cm. Preferably, it is less than 10 cm. The feed hopper may have a capacity of about 50-500 mL. Preferably, it has a capacity of below 400 mL, below, 350 mL, below 300 mL or below 250 mL.

The feed hopper is connected to a mill and configured to transfer the grain sample into the mill. It therefore further comprises a passageway that leads from the inside of the feed hopper to the mill. In some embodiments, the feed hopper is, e.g., arranged on top of the mill so that the grain filled into the feed hopper can directly fall through the passageway into the mill due to gravity. The feed hopper may however also be equipped with mechanical transporting means such as one or more conveyor belts, moving blades or threads that actively carry the grain from the feed hopper into the mill. The feed hopper can also be designed so that the user can push the grain into the mill manually after filling, e.g. with the aid of a plunger or piston. The device may alternatively also be configured to comprise and automatically move such a plunger or piston within the feed hopper to push the grain from the feed hopper into the mill.

The mill is preferably designed to comprise one or more hammer mills, and is configured to grind the grain into semolina or flour with an average particle size of less than 500 $\mu$m, preferably less than 400 $\mu$m, less than 300 $\mu$m, or less than 250 $\mu$m. In particular preferred embodiments, the mill is capable of grinding the grain into flour with an average particle size of less than 150 $\mu$m. If the mill comprises more than one hammer mill, e.g., two or three hammer mills, the different hammer mills may be preferably arranged on top of each other within the device so that the ground grain that passes the first hammer mill automatically falls into the next hammer mill below it. Repeated milling of the grain via multiple hammer mills may ensure the production of particular fine flours. Alternatively, a single hammer mill can be used, which recycles the grain until the desired grain size is reached. Various other types of mills that can be used in a device according to the invention are known to the skilled person from the prior art and include, e.g., roller mills, ball mills, disk mills or knife mills.

The mill is preferably surrounded by a suitable housing, i.e., a grinding housing, to avoid spillage of the grain prior to, during and after the milling process. The passageway of the feed hopper connects the feed hopper to the inside of the grinding housing and thus enables the grain sample to pass from the feed hopper into the mill. Preferably, the passageway is closable by any suitable mechanism known to the skilled person, e.g., via a hinged flap or a sliding plate that can be moved by the user so that the passageway is either in an open or closed state. If the passageway is closed during the grinding process, it is ensured that grain cannot exit the mill through the passageway leading back into the feed hopper.

The mill is preferably driven by a motor, e.g., an electric motor. In some embodiments, the motor can be switched on and off manually by the user, e.g., by means of a mechanical activating means such as a button or a toggle/slide switch located on an outer surface of the device. Alternatively, the motor can also be switched on via a digital user interface on a control unit connected to the device, wherein the control unit runs a software or an application suitable for controlling the motor and comprises, e.g., a touchscreen display.

In an alternative embodiment, the feed hopper or the passageway may also comprise at least one sensor which is connected to the motor of the mill via a controller such that the motor of the mill is switched on as soon as grain is introduced into the feed hopper and passes the sensor. The sensor may alternatively also be connected to the mill so that the motor is only switched on as soon as the grain from the feed hopper reaches the mill.

In alternative embodiments, it is also conceivable to drive the mill manually, e.g. by operating a crank connected to the mill.

The device according to the invention further comprises a fractionation unit, which is arranged within the device so that it can receive the ground grain directly from the mill. For instance, the fractionation unit can be arranged so that it is located below the mill, when the device is being used. Accordingly, the ground grain can directly fall from the mill into the fractionation unit due to gravity.

The fractionating unit separates the ground grain sample into at least two distinct fractions. To achieve this, the fractionation unit can be designed in different ways.

In a first embodiment, the fractionation unit comprises at least one sieve with a specific mesh size so that the ground grain is divided into at least two fractions, i.e., a first fraction consisting of particles that are sufficiently small to pass through the mesh of the sieve and a second fraction consisting of particles that are larger than the openings of the sieve and are therefore retained by the sieve.

Preferably, however, the fractionation unit comprises a plurality of sieves with different mesh sizes, e.g. two, three, four or five sieves so that the ground grain sample can be separated into more than two distinct fractions, e.g., into three, four, five or six fractions. The different sieves may be arranged so that they are located one above the other within the fractionation unit during use of the device so that the fraction passing through the upper sieve falls directly onto the next sieve below. The top sieve comprises the largest mesh size wherein the mesh sizes of the following sieves below become continuously smaller. Accordingly, the bottom sieve has the smallest mesh size of all the sieves.

To facilitate the sieving process, it is preferable for the at least one sieve to vibrate during the sieving process. The vibration can be created by the user by gently shaking the device or carefully tapping it. However, it is preferred that each of the sieves within the fractionation unit is configured so that it can be set in motion with the aid of a motor that is connected to the sieves. There are various technical ways known to the skilled person to implement this. Preferably, the sieves are flexibly and movably connected to the housing wall of the fractionation unit or the device of the invention and are configured in a way that a motor can move them either back and forth on a horizontal plane or both horizontally and vertically in a circular manner.

The motor, e.g., an electrical motor, that causes the sieves to vibrate can be switched on manually by the user via a suitable activation means or a digital user interface, as already described above for the motor that drives the mill. Alternatively, the fractionation unit can also be equipped with a sensor that switches the motor on as soon as the ground grain enters the fractionation unit and is detected by the sensor. In a further embodiment, the motor for moving the sieves can also be activated automatically at the same time or with a time delay when the motor of the mill is switched on.

In a second embodiment, the fractionation unit comprises at a triboelectric separation chamber for electrostatic fractionation of the ground grain sample. The triboelectric chamber is configured to allow the charging of the particles of the ground grain sample and is capable of applying an electric field to the charged particles to promote their fractionation based on their charge magnitude and polarity.

The design of a triboelectric separation chamber which can be used in a device according to the invention is known to the skilled person from the state of the art. For instance, triboelectric separation chambers that may serve as fractionation units in the context of the present invention are disclosed e.g. in US 6,365,856 B1, EP 0 844,026 A1 or US 3,477,568.

Irrespective of the technical implementation of the fractionation unit, the device according to the invention further comprises a fraction collector which is configured such that it receives at least one of the various fractions from the fractionation unit separately from the other fractions. Preferably, the fraction collector is configured to receive more than one fraction, most preferably each fraction from the fractionation unit separately from the other fractions. For instance, the fraction collector may comprise a plurality of chambers or pockets, wherein each of these chambers or pockets can receive and hold one fraction separately from the other fractions. If the fractionation unit has one or more sieves, the device can be designed in such a way that each individual sieve fraction is fed into a chamber or pocket of the fraction collector via a separate channel or tubing. If the fractionation unit is a triboelectric chamber, the fraction collector can also be a part of the fractionation unit. Thus, during triboelectric separation, each obtained fraction is directly fed into a chamber of the fraction collector within the fractionation unit separately from the other fractions due to its charge.

The fraction collector is further configured to feed one fraction at a time into the spectrometer for spectroscopic analysis. Preferably, the spectrometer is arranged within the device so that it is located below the fraction collector during use of the device. In some embodiments, the chambers or pockets of the fraction collector can comprise a removable base or an openable and closable opening in their base. As long as the opening in the base is closed, the fractions remain in the chambers or pockets. When these openings are opened either manually by the user or automatically, the ground grain sample falls through the bottom of its respective chamber or pocket into the sample holder of the spectrometer. The skilled person will be able to conceive of the specific ways how a fraction may be fed from the fraction collector into the spectrometer.

The spectrometer comprises a monochromator and preferably is an NIR or Raman spectrometer. It is preferably configured so that the sample holder of the spectrometer can be removed and replaced. The spectrometer preferably comprises a data memory on which the results of the various spectroscopic analyses can be stored over extended periods of time. Suitable spectrometers that can be employed in a device according to the invention are known to the skilled person.

In a preferred embodiment, the device of the present invention is further connected or connectable to a display device. The display device comprises a processor, e.g. a microcontroller, running a software or an application with a user interface. The display of the display device is preferably designed as a touchscreen.

The display device can be built into the device according to the invention, e.g. it can be part of the spectrometer.

In other embodiments, the display device is not a component of the device according to the invention, i.e., it is an external display device, e.g. a commercially available tabletop or laptop computer, a tablet or a smart phone. All of these device types are designated "computer" hereon. In these embodiments, the device of the invention is connectable to the external display device by cable or, preferably, wirelessly, e.g. by means of Bluetooth, Bluetooth Low Energy, WLAN, Near Field Communication (NFC), radio frequence (RF), radio frequency identification (RFID) or other wireless transmission methods known to the skilled person.

In some embodiments, the display device is configured to signal to the user whether a spectroscopic measurement is being performed or has been completed or failed. Preferably, the user can also directly view the results of the spectroscopic analysis for each fraction sampled via the display device. The display device can preferably also be used to start and stop the spectroscopic measurements using the device according to the invention or to alter settings and parameters of the analysis. Preferably, the display device can also be used to control other functions of the device of the invention, e.g. it can be used to activate or de-activate the motor for the mill or to control the duration of the grinding process or the coarseness setting of the mill. The display device may thus serve as the above-described control unit that controls the motor for driving the mill. Depending on how the fractionation unit of the device according to the invention is configured, the display device may also allow the user to switch the triboelectric separation chamber or the motor for moving the different sieves on or off.

The display device can also be configured to inform the user about the battery level of the device according to the invention or may issue various warnings, e.g. about the presence of contaminants or blockages in the device.

In a particularly preferred embodiment, the computer program or application installed on the display device is able to use the information from the spectroscopic analysis of at least one of the fractions of the ground grain sample to predict at least one processing property of the grain sample based on a mathematical model generated at described herein. The result of this prediction can be indicated to the user on the display device. Accordingly, in a preferred embodiment, the device according to the present invention is suitable for performing the herein disclosed method of the invention.

The invention also provides a computer program product comprising instructions which, when executed by a computer, cause the computer to carry out step d) of the method for predicting the at least one processing property of interest of the ground cereal product of the invention, i.e., to predict, on the basis of the at least one spectrum obtained in step c), the at least one processing property of interest of the ground cereal product provided in step a) using a mathematic model, wherein the mathematic model is obtainable by correlating spectra of fractions corresponding to the at least one fraction analysed in step c) from a plurality of reference ground cereal products that are different from the ground cereal product provided in step a) with the at least one processing property of interest that has been previously determined for each of the plurality of reference ground cereal products. Optionally, the computer program product is further configured to control the device of the invention.

Throughout the invention, the term "about" is intended to be understood as "+/- 10 %". If "about" relates to a range, it refers to both lower and upper limit of the range. "A" is intended to mean "one or more", if not explicitly mentioned otherwise.

All literature cited herein is herewith fully incorporated. The present invention is further illustrated, but not limited, by the following examples.

**Brief description of the drawings:**

**Figure 1:**    Schematic drawing of a device according to embodiment A comprising a fractionation unit comprising four sieves with different mesh sizes arranged one on top of the other.

**Figure 2:**   Schematic drawing of a device according to embodiment B comprising a triboelectric separation chamber as a fractionation unit.

<u>**Examples:**</u>

**Example 1**

Spectroscopy of wheat kernels and flour has been used as a rapid tool to assess wheat quality, but predictions still lack in accuracy for most quality parameters except for protein content. To enable an improved prediction of further quality characteristics, new approaches are needed. In the following, the inventors investigated if the preprocessing of flour into flour fractions and subsequent spectroscopic analysis of these fractions could be a new way to improve wheat quality predictions. The following is an example of the results of a multi-year trial in which the method of the present invention was used to determine the baking properties of flour.

**1. Materials and Methods**

1.1 Flour samples

50 commercially available wheat flour samples, representing mixtures of wheat cultivars, were used. 27 samples originated from Germany (harvest years 2019-2022), 8 from Australia (harvest years 2020-2021), 5 from USA (harvest year 2021), 2 from Lativa (harvest year 2021), 2 from Lithuania (harvest years 2020-2021), 2 from Mexico (harvest year 2021), 1 from India (harvest year 2021), 1 from Poland (harvest year 2021), 1 from Romania (harvest year 2021) and 1 from Ukraine (harvest year 2021). Wheat classes were mostly unknown, but samples included a range of different qualities (e.g. German quality E, A and B, Australian Hard, Australian Prime Hard, Australian Premium White, Hard red spring and Hard red winter).
After milling, flour samples had ash contents of approximately 0.60 % and protein contents ranged from 9.2 - 16.5 %.

1.2 Rheological tests

Flour rheological analyses included Farinograph measurements (according to ICC 115), Extensograph measurements (according to ICC114/1), AlveoLab measurements (according to ICC 121) and MixoLab measurements (according to ICC 173).
The following parameters were obtained and used for modeling:

- Farinograph: dough development time DDT; consistency C; water absorption (WZ, WAC, WAM); stability S; dough softening DS (10 min after start, 12 min after DDT); quality number FQN

- Extensograph: water absorption; energy (45, 90, 135 min); resistance (45, 90, 135 min); extensibility (45, 90, 135 min); maximum (45, 90, 135 min); ratio number (45, 90, 135 min); ratio number max. (45, 90, 135 min)

- AlveoLab: maximum pressure Cmax; tenacity P; extensibility L; swelling index G; deformation energy W; ratio P/L; elasticity index le; strength coefficient K; strain hardening index SH; minimum of first derivative Dmin; maximum of first derivative Dmax

- MixoLab: water absorption; dough development time; stability; torques (C1; CS; C2; C3; C4; C5)

1.3 Flour fractionation and dough preparation

Flours were fractionated by different methods, namely by air-jet sieving and preparation of dough that was washed out to obtain gluten and starch.
For air-jet sieving, 70 g of each flour sample was air jet sieved (e.g. using the 200LS-N Hosokawa Alpine AG) using a 32 $\mu$m, 50 $\mu$m, 75 $\mu$m and 100 $\mu$m sieve. Sieving started with the smallest sieve. After 10 min, the remaining material on the sieve was transferred to the next larger sieve. In this way, the following five sieve fractions were obtained: < 32 $\mu$m, 32-50 $\mu$m, 50-75 $\mu$m, 75-100 $\mu$m, > 100 $\mu$m.
For washing, dough was prepared in a farinograph according to ICC 115, but only 30 g of flour was used and 0.60 g of salt was added. Dough was mixed until dough development time was reached that had been determined prior to dough preparation also according to ICC 115. Then, two times 15 g of dough were washed out using a Glutomatic device. The remaining dough as well as the glutens after the washing procedure were covered and rested for 10 min and afterwards frozen at -28 °C. This process was repeated three times for each flour sample. Then, the obtained starch slurry was centrifuged and the precipitate also frozen at -28 °C. The next day, frozen dough, gluten and starch samples were freeze-dried for 24 h. Freeze-dried samples were milled to powders using an Ultra Centrifugal Mill.

1.3 Spectroscopic analyses

1.3.1 Fluorescence spectroscopy

Two-dimensional fluorescence spectra were recorded using the BioView® Sensor (Delta Light & Optics). Excitation was scanned between 270-550 nm and emission between 310-590 nm using 20 nm increments each. Each sample was placed in a petri dish (QJ 6 cm) and evenly compacted before fluorescence spectra were taken at six different positions.

1.3.2 Near Infrared (NIR) spectroscopy

Near-infrared spectra were recorded using the MPA (Bruker Optik GmbH). Diffuse reflectance measurements were carried out using a rotating cup. A resolution of 8 $cm^{-1}$ and 64 scans per measurement were used to obtain six absorbance spectra of every sample. In between measurements, samples were thoroughly mixed.

1.3.3 Raman spectroscopy

Raman spectra were recorded using the Raman 785 (inno-spec GmbH) and a fiber optic probe. The used laser wavelength was 785 nm. Integration time and number of scans were chosen separately for flour and fractions in order to achieve a high signal-to-noise ratio. 9 s and 12 scand were used for starch samples, 2.5 s and 50 scans for gluten samples and 6.5 s and 15 scans for flour and sieve fractions. Each sample was placed in a petri dish (QJ 6 cm) and evenly compacted before Raman spectra were taken at six different positions.

1.4 Baking test

A mini-baking test was used to assess the baking quality of the flour samples. In a Farinograph (63 rpm, 27 °C), 160 g of flour (14 % moisture), 1.6 g of dry yeast, 1.6 g of sugar, 1.6 g of fat and 2.4 g of salt were mixed for 1 min. Then, an ascorbic acid solution was added (0.0032 g dissolved in a variable amount of water for every flour samples WAM, determined by ICC 115). The dough was then kneaded until the dough development time was reached (also determined according to ICC 115). The dough was placed in a proofing chamber (32 °C, 80 % rel. humidity) for 30 min minus the dough development time. Then, 4 x 60 g of dough were mechanically rounded, placed in baking rings on a baking tray and then back in the proofing chamber for 70 min minus the time it took to round the dough pieces. The breads were baked for 20 min at 230 °C and then cooled for 1 h at room temperature before bread weights and volumes were determined.

1.5 Chemometric analyses

Prediction models for specific bread volume were calculated based on spectral and rheological data using principal component regression (PCR) and partial least squares regression (PLSR). A leave-one-out cross-validation (LOOCV) was performed. Best models were determined by the minimum root mean square error of cross-validation (RMSECV). Additionally, the coefficient of determination of cross-validation ($R^2_{CV}$) was calculated.

$$R^2_{CV} = 1 - \frac{\sum_{i=1}^{n}(y_i - \widehat{y_i})^2}{\sum_{i=1}^{n}(y_i - \bar{y})^2}$$

$$RMSECV = \sqrt{\frac{\sum_{i=1}^{n}(y_i - \widehat{y_i})^2}{n}}$$

n = total number of samples

$y_i$ $\widehat{y_i}$ = measured value of i'th sample = predicted value of i'th sample

$\bar{y}$ = mean value of sample set

Spectra were preprocessed using common algorithms, including e.g. Savitzky-Golay smoothing and differentiation filters, highpass filter, iterative polynomial baseline removal, detrending, standard normal variate transformation and (extended) multiplicative scatter correction as well as normalizations (min-max, 1. norm, 2. norm). When models were based on a combination of spectra, spectral intensities of the respective fractions and potentially flour spectra were concatenated before the regression was carried out. In case spectral intensities were combined with rheological measurements, autoscaling of variables was performed to account for different units before spectra and rheological parameters. Then, spectra were concatenated with all rheological parameters of a particular rheological test (described in section 1.2) and used for regression modeling.

## 2. Results and Discussion

In the following, it is shown how the prediction of the baking volume can be improved by analysing different flour fractions as well as combinations of fractions using different spectroscopic methods and by taking the rheological properties of dough prepared from the tested flours into account.

*2.1 Improving the prediction of baking volume by spectroscopic analysis of flour fractions rather than whole flour*

The inventors first tested the predictive power with regard to the baking property "baking volume" by analysing thefluorescence, NIR or Raman spectra of unfractionated flour. Subsequently, it was tested whether the spectroscopic analysis of individual flour fractions obtained by different methods can significantly improve the prediction of baking volume compared to the spectroscopic analysis of unfractionated flour samples. The results of these experiments are summarized in Table 1:

Table 1: Cross-validation results for prediction of specific bread volume from fluorescence, NIR and Raman spectra using PCR or PLSR and individual spectra of flour or fractions.

| Sample | Fluorescence | | NIR | | Raman | |
|---|---|---|---|---|---|---|
| | $R^2_{CV}$ | RMSECV [mL/g bread] | $R^2_{CV}$ | RMSECV [mL/g bread] | $R^2_{CV}$ | RMSECV [mL/g bread] |
| **Unfractionated Flour** | 0.71 | 0.27 | 0.85 | 0.20 | 0.81 | 0.22 |
| **Gluten** | 0.81 | 0.22 | 0.59 | 0.32 | 0.58 | 0.33 |
| **Starch** | 0.52 | 0.35 | 0.69 | 0.28 | 0.64 | 0.30 |
| **Dough** | 0.71 | 0.27 | 0.84 | 0.20 | 0.84 | 0.21 |
| **< 32 $\mu$m** | 0.57 | 0.33 | 0.72 | 0.27 | 0.78 | 0.24 |
| **32-50 $\mu$m** | 0.66 | 0.30 | 0.81 | 0.23 | 0.76 | 0.25 |
| **50-75 $\mu$m** | 0.70 | 0.28 | 0.82 | 0.21 | 0.85 | 0.20 |
| **75-100 $\mu$m** | 0.74 | 0.26 | 0.85 | 0.20 | 0.85 | 0.20 |
| **> 100 $\mu$m** | 0.68 | 0.29 | 0.83 | 0.21 | 0.84 | 0.20 |

As can be seen in Table 1, baking volume could be predicted from fluorescence spectra of unfractionated flour with a coefficient of determination $R^2$ of 0.71 and a prediction error of 0.27 mL/g bread.

By fractionating the flour to gluten (dough preparation in the Farinograph according to ICC 115, leaching of the dough according to ICC 155, freeze-drying, grinding) and subsequent recording of the gluten spectra, the prediction could be improved to $R^2$ = 0.81 with a prediction error of 0.22 mL/g bread.

Similarly, fractionation of the flour into sieve fractions (air jet sieving using sieve sizes 32 $\mu$m, 50 $\mu$m, 75 $\mu$m, 100 $\mu$m) and subsequent analysis of the fluorescent spectra of the 75-100 $\mu$m fraction also slightly improved the prediction to $R^2$ = 0.74 with a prediction error of 0.26 mL/g bread.

Also the analysis of individual flour fractions using Raman spectroscopy could improve the prediction of bread volume.

As can be seen in Table 1, the specific bread volume could be predicted from the Raman spectra of unfractionated flour with $R^2$ = 0.81 and a prediction error of 0.22 mL/g bread.

If the flours were previously processed into sieve fractions (air jet sieving using sieve sizes 32 $\mu$m, 50 $\mu$m, 75 $\mu$m, 100 $\mu$m), an improved prediction of the specific bread volume could be achieved. In particular, it was possible to achieve a prediction improvement to $R^2$ = 0.85 and a prediction error of 0.20 mL/g bread from the spectra of the 50-75 $\mu$m fraction as well as from the spectra of the 75-100 $\mu$m fraction.

Accordingly, at least for fluorescence spectroscopy and Raman spectroscopy, improvements in the prediction of the specific bread volume could be achieved by analysing individual flour fractions rather than unfractionated flour.

Based on the results in Table 1, the inventors further investigated whether the prediction of the baking volume of flour could be further improved by combining the spectroscopic measurements of several different fractions. The results of these investigations are summarized in Table 2.

Table 2: Cross-validation results for prediction of specific bread volume from fluorescence, NIR and Raman spectra using PCR or PLSR and a combination of two or three spectra.

| Spectroscopy | Combination | | $R^2_{CV}$ | RMSECV [mL/g bread] |
|---|---|---|---|---|
| Fluorescence | 2 Spectra | < 32 $\mu$m+50-75 $\mu$m | 0.75 | 0.26 |
| NIR | 2 Spectra | Gluten+Flour | 0.88 | 0.18 |
| | | Flour+Starch | 0.87 | 0.18 |
| | | 32-50 $\mu$m + 75-100 $\mu$m | 0.87 | 0.18 |
| | | < 32 $\mu$m + > 100 $\mu$m | 0.86 | 0.19 |
| | 3 Spectra | Gluten+Flour+Starch | 0.91 | 0.15 |
| Raman | 2 Spectra | < 32 $\mu$m + 75-100 $\mu$m | 0.91 | 0.15 |
| | | < 32 $\mu$m + 50-75 $\mu$m | 0.88 | 0.18 |
| | | < 32 $\mu$m + > 100 $\mu$m | 0.87 | 0.18 |
| | | 50-75 $\mu$m + > 100 $\mu$m | 0.87 | 0.18 |
| | | < 32 $\mu$m + Flour | 0.86 | 0.19 |

Indeed, for fluorescence spectroscopy, a combination of the < 32 $\mu$m fraction with the 50-75 $\mu$m fraction obtained by air jet sieving achieved an improved prediction of baking volume ($R^2$ = 0.75, prediction error 0.26 mL/g bread) than when only the individual 50-75 $\mu$m flour fraction was analysed ($R^2$ = 0.70, prediction error 0.28 mL/g bread, cf. Table 1). An analysis of at least two size fractions, optionally, including a 50-75 $\mu$m fraction and a smaller fraction, e.g., a < 32 $\mu$m fraction is thus preferred in the method of the invention, in particular, if analysed by fluorescence spectroscopy.

For NIR spectroscopy, the specific bread volume could be predicted from the NIR spectra of unfractionated flour with $R^2$ = 0.85 and a prediction error of 0.20 mL/g bread (Table 1). As can be seen in Table 2, by fractionating the flour to gluten and starch (dough preparation in the Faringograph according to ICC 115, washing of the dough according to ICC 155, freeze-drying, grinding) and subsequent recording of the spectra of gluten and starch, the prediction could be improved by combining flour spectra with gluten and starch spectra to $R^2$ = 0.91 and a prediction error of 0.15 mL/g bread. Fractionation into sieve fractions (air jet sieving using sieve sizes 32 $\mu$m, 50 $\mu$m, 75 $\mu$m, 100 $\mu$m) and subsequent recording and combination of the NIR spectra of the 32-50 $\mu$m and 75-100 $\mu$m fractions also improved the prediction to $R^2$ = 0.87 and a prediction error of 0.18 mL/g bread. An analysis of at least two size fractions, optionally, including a 75-100 $\mu$m fraction and a smaller fraction, e.g., a 32-50 $\mu$m fraction is thus preferred in the method of the invention, in particular, if analysed by NIR spectroscopy.

Finally, for Raman spectroscopy, if the spectra of the < 32 $\mu$m and 75-100 $\mu$m fractions were combined, the $R^2$ could be improved to 0.91 and the prediction error to 0.15 mL/g bread compared to the prediction based on the unfractionated flour samples ($R^2$ = 0.81, prediction error of 0.22 mL/g bread, cf. Table 1). Also the combination of the spectra of the < 32 $\mu$m and 50-75 $\mu$m fractions ($R^2$ = 0.88, prediction error of 0.18 mL/g bread), the combination of the spectra of the < 32 $\mu$m and the > 100 $\mu$m fractions ($R^2$ = 0.87, prediction error of 0.18 mL/g bread) and the combination of the spectra of the 50-75 $\mu$m and > 100 $\mu$m fractions ($R^2$ = 0.87, prediction error of 0.18 mL/g bread) significantly improved the prediction of the baking volume. An analysis of at least two size fractions, including a 75-100 $\mu$m or 50-75 $\mu$m fraction and a smaller fraction, e.g., a <32 $\mu$m fraction is thus preferred in the method of the invention, in particular, if analysed by Raman spectroscopy.

*2.2 Improving the prediction of baking volume supplementing the spectroscopic analysis of of flour samples with rheological measurements.*

By supplementing the fluorescence spectra of unfractionated flour with rheological Extensograph or Farinograph data, the prediction of the specific baking volume of flours could be improved from $R^2$ of 0.71 and a prediction error of 0.27 mL/g bread (cf. Table 1) to $R^2$ = 0.86 or $R^2$ = 0.80 and a prediction error of 0.20 or 0.22 mL/g bread. The inclusion of more than one rheological test led to a further improvement in prediction. For example, a model based on flour spectra combined with Extensograph and MixoLab data achieved an $R^2$ = 0.88 and a prediction error of 0.18 mL/g bread.

Similarly, also the combination of NIR whole flour spectra with rheological analyses also led to a significant improvement in prediction. The specific bread volume could be predicted from the NIR whole flour spectra with $R^2 = 0.85$ and a prediction error of 0.20 mL/g bread (Table 1). By supplementing the flour spectra with Farinograph or Extensograph measurements, an $R^2$ value of 0.91 and prediction errors of 0.15 and 0.16 mL/g of bread respectively were achieved. A further improvement was achieved when flour spectra were combined with Farinograph and AlveoLab data. The $R^2$ thus improved to 0.92 and the prediction error to 0.14 mL/g bread.

Finally, by supplementing the Raman spectra of unfractionated flour with Farinograph and Extensograph measurements, the prediction of the specific bread volume improved from $R^2 = 0.81$ and a prediction error of 0.22 mL/g bread (Table 1) to $R^2 = 0.84$ and $R^2 = 0.87$ and the prediction error to 0.20 and 0.19 mL/g bread respectively.

Thus, these results clearly demonstrate that by expanding flour spectroscopy using rheological investigations, it is possible to improve predictions for fluorescence, NIR and Raman spectroscopy.

**Example 2**

In the following example two distinct embodiments of the portable device according to the invention, referred to as embodiments A and B, respectively, are described in more detail.

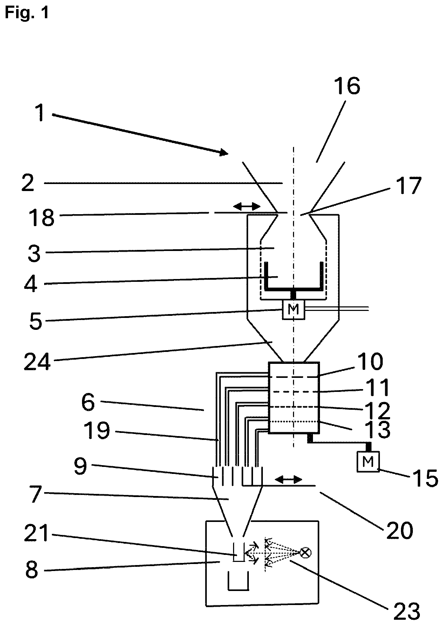

A schematic of a portable device (1) according to embodiment A is presented in Figure 1.

The portable device (1) comprises a housing (24) and a feed hopper (2) for receiving the grain. The feed hopper can be filled manually by the user. The feed hopper (2) should comprise an opening (16) that is sufficiently large to receive the grain. The diameter of this opening (16) of the feed hopper (2) can be about 5-20 cm. Preferably, it is less than 10 cm. The feed hopper (2) may have a capacity of about 50-500 mL. Preferably, it has a capacity of below 400 mL, below, 350 mL, below 300 mL or below 250 mL.

The feed hopper (2) is connected to a grinding housing (3) comprising a mill (4), which may be designed, e.g., as a hammer mill. The feed hopper (2) is thus configured to transfer the grain sample directly into the mill (4) within the grinding housing (3). The grinding housing (3) avoids spillage of the grain prior to, during and after the milling process. Moreover, optionally, the passageway (17) that connects the feed hopper (2) to the inside of the grinding housing (3) and thus enables the grain sample to pass from the feed hopper (2) into the mill (4) can be opened or closed, e.g., using a sliding plate (18) that can be moved by the user back and forth so that the passageway (17) is either in an open or closed state. If the passageway is closed during the grinding process, it is ensured that grain cannot exit the mill through the passageway leading back into the feed hopper.

As can be further seen in Figure 1, the mill (4) is connected to a motor (5), e.g. an electric motor, that drives the mill (4). The motor (5) of the mill (4) can be inside the grinding housing (3) or outside of it. In some embodiments, the motor (5) can be switched on and off manually by the user, e.g., by means of a mechanical activating means such as a button or a toggle/slide switch located on the surface of the device. Alternatively, the motor (5) can also be switched on via a digital user interface on a control unit connected to the device, wherein the control unit runs an application suitable for controlling the motor and comprises, e.g., a touchscreen display.

In an alternative embodiment, the feed hopper (2) or the passageway (17) may also comprise at least one sensor which is connected to the motor (5) of the mill (4) via a controller such that the motor (5) of the mill (4) is switched on as soon as the grain is introduced into the feed hopper (2) and passes the sensor. To save energy, the sensor may alternatively also be connected to the mill (4) so that the motor (5) is only switched on as soon as the grain from the feed hopper (2) reaches the mill (4).

The device according to the invention further comprises a fractionation unit (6) located below the mill (4), which is configured to receive the ground grain sample from the mill (4). The fractionating unit (6) is configured to separate the ground grain sample into at least two distinct fractions.

As can be seen in Figure 1, in embodiment A, the fractionation unit (6) comprises a plurality of vibrating sieves (10, 11, 12, 13) arranged one above the other for mechanical fractionation of the ground grain sample into five distinct fractions based on particle size. The top sieve (10) comprises the largest mesh size. The second sieve (11) arranged underneath the top sieve has a smaller mesh size than the top sieve (10). The third sieve (12) underneath the second sieve (11) has a mesh size smaller than that of the top and the second sieves (10, 11). Finally, the bottom sieve (13) has the smallest mesh size of all the sieves.

The sieves (10, 11, 12, 13) vibrate during the sieving process because they are moved back and forth by a motor (15) connected to the sieves (10, 11, 12, 13).

The motor (15) that causes the sieves (10, 11, 12, 13) to vibrate can, in some embodiments, be switched on manually by the user via a suitable activation means or a digital user interface, as already described above for the motor (5) that drives the mill (4). Alternatively, the fractionation unit (6) can also be equipped with a sensor that switches the motor (15) on as soon as the ground grain enters the fractionation unit (6) and is detected by the sensor. In a further embodiment, the motor (15) for moving the sieves (10, 11, 12, 13) can also be activated automatically at the same time or with a time delay when the motor (5) of the mill (4) is switched on.

The device (1) according to the invention further comprises a fraction collector (7) which is configured such that it receives at least one of the fractions from the fractionation unit (6) separately from the other fractions. As can be seen in Figure 1, the fraction collector comprises a plurality of chambers (9), wherein each of these chambers (9) can receive and hold one fraction separately from the other fractions. The device (1) is designed in such a way that each individual sieve fraction is fed into a chamber (9) of the fraction collector (7) via a separate channel (19).

The fraction collector (7) is further configured to feed one fraction at a time into a spectrometer (8) joint to the fraction collector (7) for spectroscopic analysis. As can be seen in Figure 1, the chambers (9) of the fraction collector (7) may have a removable base (20). As long as the removable base (20) is closed, the fractions remain in the chambers (9). When the removable base (20) of a chamber (9) is removed, e.g., by sliding the base horizontally, the ground grain sample falls through the bottom of its respective chamber (9) into the sample holder (21) of the spectrometer (8).

The spectrometer (8) comprises a monochromator (23) and can be an NIR or Raman spectrometer. It is preferably configured so that the sample holder (21) of the spectrometer (8) can be removed and replaced. The spectrometer (8) preferably comprises a data memory on which the results of the various spectroscopic analyses can be stored over extended periods of time.

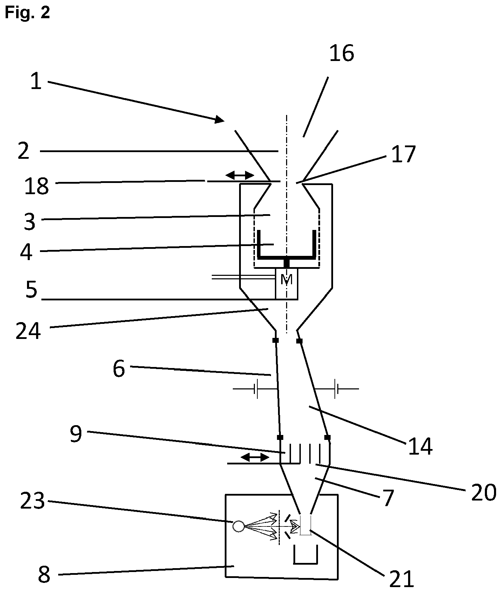

Embodiment B of the device (1) according to the invention is almost identical to embodiment A described above. As can be seen in Figure 2, embodiment B differs from embodiment A merely in that the fractionation unit (6), which receives the ground grain sample from the mill (4) inside the grinding housing (3) comprises a triboelectric separation chamber (14) for electrostatic fractionation of the ground grain sample. The triboelectric separation chamber (14) is configured to allow the charging of the particles of the ground corn sample and is capable of applying an electric field to the charged particles to promote their fractionation based on their charge magnitude and polarity.

The design of a triboelectric separation chamber (14) which can be used in a device (1) according to the invention is known to the skilled person from the state of the art. For instance, triboelectric separation chambers that may serve as fractionation units in the context of the present invention are disclosed e.g. in US 6,365,856 B1, EP 0 844,026 A1 or US 3,477,568.

In embodiment B, too, the different fractions obtained by electrostatic fractionation are first collected separately in the fraction collector (7) and then fed to the spectrometer (8) to be analysed spectroscopically.

Irrespective of whether the fractionation unit (6) is configured as described for embodiment A in Figure 1 or embodiment B in Figure 2, the device (1) of the present invention is further connected or connectable to a display device (22), which is not shown in Figures 1 or 2. The display device (22) can be built into the device (1) according to the invention, e.g. it can be part of the spectrometer (8). The display device (22) comprises a processor, e.g. a microcontroller, on which software or an application with a user interface is installed. The display of the display device is preferably designed as a touchscreen.

In other embodiments, the display device (22) is not a component of the device according to the invention, i.e., it is an external display device, e.g. a commercially available computer, a tablet or a smart phone. In these embodiments, the device of the invention is connectable to the external display device by cable or, preferably, wirelessly, e.g. by means of Bluetooth, Bluetooth Low Energy, WLAN, Near Field Communication (NFC), radio frequence (RF), radio frequency identification (RFID) or other wireless transmission methods known to the skilled person.

In some embodiments, the display device (22) is configured to signal to the user whether a spectroscopic measurement is being performed or has been completed or failed. Preferably, the user can also directly view the results of the spectroscopic analysis for each fraction sampled via the display device (22). The display device (22) can preferably also be used to start and stop the spectroscopic measurements using the device according to the invention or to alter settings and parameters of the analysis. Preferably, the display device (22) can also be used to control other functions of the device (1) of the invention, e.g. it can be used to activate or de-activate the motor (5) for the mill (4) or to control the duration of the grinding process or the coarseness settings of the mill (4). The display device (22) may thus serve as the above-described control unit that controls the motor (5) for driving the mill (4). The display device (22) may also allow the user to switch the motor (15) for moving the different sieves (10, 11, 12, 13) on or off in embodiment A. The display device (22) may also be used to control the triboelectric separation chamber (14) in embodiment B.

The display device (22) can also be configured to inform the user about the battery level of the device (1) according to the invention or may issue various warnings, e.g. about the presence of contaminants or blockages in the device (1).

In a particularly preferred embodiment, the software or application installed on the display device (22) is able to use the information from the spectroscopic analysis obtained from the spectrometer (8) of at least one of the fractions of the ground grain sample to predict, preferably, in real time, at least one processing property of the grain sample based on a mathematical model generated as described herein. The result of this prediction can be indicated to the user on the display device (22). Accordingly, in a preferred embodiment, the device (1) is suitable for performing the herein disclosed method of the invention.

**References:**

Pojić, M. M., and Mastilović, J. S., 2013. Near Infrared Spectroscopy-Advanced Analytical Tool in Wheat Breeding, Trade, and Processing. Food Bioproc. Technol. 6(2), 330-352. https://doi.org/10.1007/s11947-012-0917-3.

Nagel-Held, J., Kaiser, L., Longin, C. F. H., and Hitzmann, B., 2022. Prediction of wheat quality parameters combining Raman, fluorescence, and near-infrared spectroscopy (NIRS). Cereal Chem. 99(4), 830-842. https://doi.org/10.1002/cche.10540

Lakowicz, J., 2006. Principles of Fluorescence Spectroscopy. Principles of Fluorescent Spectroscopy, 3rd Edn, 1. https://doi.org/10.1007/978-0-387-46312-4

Osborne, B.G., 1984. Investigations into the use of near infrared reflectance spectroscopy for the quality assessment of wheat with respect to its potential for bread baking. J. Sci. Food Agric. 35(1), 106-110.

Ahmad, M. H., Nache, M., Waffenschmidt, S., and Hitzmann, B., 2016. Characterization of farinographic kneading process for different types of wheat flours using fluorescence spectroscopy and chemometrics. Food Control 66, 44-52. https://doi.org/10.1016/j.foodcont.2016.01.029 Nawrocka, A., Miś, A., and Szymańska-Chargot, M., 2016. Characteristics of Relationships Between Structure of Gluten Proteins and Dough Rheology - Influence of Dietary Fibres Studied by FT-Raman Spectroscopy. Food Biophys. 11(1), 81-90. https://doi.org/10.1007/s11483-015-9419-y

Rubenthaler, G.L., and Pomeranz, Y., 1987. Near-infrared reflectance spectra of hard red winter wheats varying widely in protein content and breadmaking potential. Cereal Chem. 64(6), 407-411.

Delwiche, S.R., and Weaver, G., 1994. Bread quality of wheat flour by near-infrared spectrophotometry: Feasibility of modelling. J. Food Sci. 59(2), 410-415.

Bengtsson, S., 2002. NIR determination of flour baking properties.

WO 2002/054061 A1.

Sorvaniemi, J., Kinnunen, A., Tsados, A., and Mälkki, Y., 1993. Using partial least squares regression and multiplicative scatter correction for FT-NIR data evaluation of wheat flours. Lebensm. Wiss. Technol. 26, 251-258.

Nagel-Held, J., El Hassouni, K., Longin, F., and Hitzmann, B., 2024. Spectroscopy-based prediction of 73 wheat quality parameters and insights for practical applications. Cereal Chem. 101, 144-165. https://doi.org/10.1002/cche.10732 en.wikipedia.org/wiki/Semolina

Sacchetti, G., Coccob. G., Coccob, D., Neria, L., and Mastrocola, D., 2011. Effect of semolina particle size on the cooking kinetics and quality of spaghetti. Proc. Food Sci. 1, 1740-1745. www.haverparticleanalysis.com/en/sieve-analysis/ro-tapr-test-sieve-shaker/    www.retsch.com/products/sieving/sieve-shakers/as-200-control/function-features/    gkm-net.de/en/laboratory-air-jet-lab-sieves.html

Hareland, 1994. Evaluation of flour particle size distribution by laser diffraction, sieve analysis and near-infrared reflectance spectroscopy. J. Cereal Sci. 20(2), 183-190.

Žilić, S., Barać, M., Pešić, M., Dodig, D., and Ignjatović-Micić, D., 2011. Characterization of proteins from grain of different bread and durum wheat genotypes. Int. J. Mol. Sci. 12(9), 5878-5894.

Shewry, P. R., Halford, N. G., Belton, P. S., and Tatham, A. S., 2002. The structure and properties of gluten: an elastic protein from wheat grain. Phil. Trans. R. Soc. Lond. 357, 133-142.

https://de.wikipedia.org/wiki/Fallzahl

Czaja T, Sobota A, and Szostak R, 2020. Quantification of Ash and Moisture in Wheat Flour by Raman Spectroscopy. Foods 9(3), 280. doi: 10.3390/foods9030280. PMID: 32138384; PMCID: PMC7143060.

Jensen, S. A., Munck, L., and Martens, H., 1982. The Botanical Constituents of Wheat and Wheat Milling Fractions. Quantification by Autofluorescence. Cereal Chem. 59(6), 477-484. Retrieved from https://www.cerealsgrains.org/publications/cc/backissues/1982/Documents/chem59_477.pdf Jones, C. R., Halton, P., and Stevens, D. J., 1959. The separation of flour into fractions of different protein contents by means of air classification. J. Biochem. Microbiol. Technol. Eng. 1(1), 77-98. https://doi.org/10.1002/jbmte.390010108

Youssef, M. M., and Abo-Dief, M. F., 2022. Air Classification: Eco-Friendly Separation Technique in Food Technology- An Article. Alexandria J. Food Sci. Technol. 19(1), 10-14. https://doi.org/10.21608/ajfs.2022.255779

https://en.wikipedia.org/wiki/Air_classifier

Whitelaw, W., 2002. Particle separator and method of separating particles. US 6,365,856 B1. Geisler, I., Kauer, H.J., and Stahl, I., 1998. Electrostatic separation apparatus for separating triboelectric charged mixtures of material. EP 0 844 026 A1.

Robert, M., 1969. Electrostatic separation of round and nonround particles. US 3,477,568.

**Claims**

1. A method for predicting at least one processing property of interest of a ground cereal product, comprising steps of:

a) providing a ground cereal product;

b) separating the ground cereal product into at least two distinct fractions according to at least one parameter;

c) analysing at least one of these fractions by spectroscopy to obtain at least one spectrum, wherein optionally, at least two fractions are analysed; and

d) on the basis of the at least one spectrum obtained in step c), predicting the at least one processing property of interest of the ground cereal product provided in step a) using a mathematic model, wherein the mathematic model is obtainable by correlating spectra of fractions corresponding to the at least one fraction analysed in step c) from a plurality of reference ground cereal products that are different from the ground cereal product provided in step a) with the at least one processing property of interest that has been previously determined for each of the plurality of reference ground cereal products;

wherein the at least one processing property is a baking property of the ground cereal product or a rheological property of dough prepared from the ground cereal product.

2. The method of claim 1, wherein the parameter according to which the ground cereal product is separated into the at least two fractions is based on particle size, particle density and/or particle shape, preferably particle size.

3. The method of any of the preceding claims, wherein separation of the ground cereal product into at least two distinct fractions is achieved via a method selected from the group consisting of sieving, air classification, and tribo-electrostatic separation.

4. The method of claim 3, wherein separation of the ground cereal product into at least two distinct fractions is achieved via sieving, preferably via air-jet sieving.

5. The method of any of the preceding claims, wherein the spectroscopy is selected from the group consisting of NIR spectroscopy, fluorescence spectroscopy and Raman spectroscopy.

6. The method of any of the preceding claims, wherein the at least one processing property of the ground cereal product is a baking property selected from the group comprising baking volume, bread texture, bread colour, water absorption, and flour falling number, preferably, baking volume.

7. The method of any of claims 1-4, wherein the at least one processing property is a rheological property selected from the group comprising dough extensibility, dough stretch resistance, and dough kneading tolerance.

8. The method of any of claims 1-6, wherein the processing property of interest is a baking property and wherein the method further comprises:

I. measuring at least one rheological property of dough prepared from the ground cereal product provided in step a) of claim 1;

II. predicting the at least one baking property of the ground cereal product provided in step a) of claim 1 using the mathematical model, wherein the mathematical model is further based on the correlation of at least one rheological property of dough produced from each of the plurality of reference ground cereal products with the at least one baking property of interest that has been previously determined for each of the plurality of reference ground cereal products.

9. The method of any of the preceding claims, wherein the mathematical model is obtainable by the steps of:

i. providing a plurality of reference ground cereal products that are different from the ground cereal product provided in step a) of claim 1;

ii. determine for each of the plurality of reference ground cereal products the at least one processing property of interest using standardized tests;

iii. separating each of the plurality of reference ground cereal products into at least two distinct fractions that correspond to the at least two fractions obtained in step b) of claim 1;

iv. analysing at least one of the fractions of each of the plurality of reference ground cereal products by spectroscopy to obtain at least one spectrum, wherein optionally, at least two fractions are analysed in step iv);

v. optionally, measuring for dough prepared from each of the plurality of reference ground cereal products at least one rheological property, wherein the at least one rheological property corresponds to the rheological property measured in step I of claim 8;

vi. generating the mathematical model by correlating, for each of the plurality of reference ground cereal products, the results of the spectroscopic analysis obtained in step iv and, optionally, the measurements of step v with the at least one processing property determined in step ii.

10. The method of any of the preceding claims, wherein the ground cereal product is flour, semolina or a mixture thereof.

11. The method of any of the preceding claims, wherein the cereal is wheat selected from the group comprising *Triticum aestivum*, *Triticum durum, Triticum dicoccum, Triticum spelta, Triticum monococcum* and a mixture thereof, wherein the wheat preferably is *Triticum aestivum.*

12. A device (1) for the preparation and spectroscopic analysis of grain samples, comprising:

- a feed hopper (2) for receiving a grain sample and
- a mill (4), wherein the feed hopper (2) is configured to transfer the grain sample into the mill (4), wherein the mill (4) is configured to grind the grain sample,
- a fractionation unit (6), which is configured to receive the ground grain sample from the mill (4) and to separate the ground grain sample into at least two different fractions,
- a fraction collector (7), which is configured to receive and store at least one of the fractions of the ground grain sample separately from other fractions from the fractionation unit (6), and
- a spectrometer (8), which is joined to the fraction collector (7) and is configured to receive and analyse the at least one fraction separately from other fractions.

13. The device (1) of claim 12, wherein the fractionation unit (6) comprises

a) one or more vibrating sieves (10, 11, 12, 13) for mechanical fractionation of the ground grain sample, or
b) a triboelectric separation chamber (14) for electrostatic fractionation of the ground grain sample,

preferably, one or more vibrating sieves (10, 11, 12, 13) for mechanical fractionation of the ground grain sample.

14. The device (1) of any of claims 12 or 13, wherein the device (1) is further connected or connectable to a display device (22) comprising a processor running a computer program with a user interface, wherein, optionally, the device is suitable for performing the method of any of claims 1-12.

15. A computer program product comprising instructions which, when executed by a computer, cause the computer to carry out step d) of claim 1 in the method for predicting the at least one processing property of interest of the ground cereal product of any of claims 1-11, wherein, optionally, the computer program product is further configured to control the device of any of claims 12-14.

**Fig. 1**

**Fig. 2**

Europäisches Patentamt

European Patent Office

Office européen des brevets

# EUROPEAN SEARCH REPORT

**Application Number**

EP 24 19 0422

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | NAGEL-HELD JOHANNES ET AL: "Prediction of wheat quality parameters combining Raman, fluorescence, and near-infrared spectroscopy (NIRS)", CEREAL CHEMISTRY, vol. 99, no. 4, 14 March 2022 (2022-03-14), pages 830-842, XP093234056, US ISSN: 0009-0352, DOI: 10.1002/cche.10540 | 1-10, 13-15 | INV. G01N21/85 G01N21/359 G01N21/3563 G01N21/64 G01N21/65 B03C7/00 G01N33/10 |
| Y | * abstract; tables 1-4 * * page 832, paragraph 2.1 - page 841, paragraph 4 * | 11,12 | ADD. G01N21/84 G01N15/00 G01N1/00 |
| Y | JP 2 892084 B2 (SATAKE ENG CO LTD) 17 May 1999 (1999-05-17) * figure 1 * | 12 | |
| Y | US 2023/329290 A1 (POPPER LUTZ [DE] ET AL) 19 October 2023 (2023-10-19) | 11 | |
| A | * paragraphs [0001], [0029]; claim 11 * | 3,13 | |
| A | US 2016/341649 A1 (KITTELSON JAYD MARSHAL [US]) 24 November 2016 (2016-11-24) * paragraphs [0002], [0003], [0058], [0066] - [0069]; figures 5A-5C * | 1 | TECHNICAL FIELDS SEARCHED (IPC)<br><br>B03C<br>G01N |
| A | CN 207 571 113 U (ROLANDS VINOGRADOVS; BALTIC AGRO TEHNOLOGIJAS SIA) 3 July 2018 (2018-07-03) * paragraph [0001]; claims 1,7; figure 1 * | 1 | |

The present search report has been drawn up for all claims

| Place of search | Date of completion of the search | Examiner |
|---|---|---|
| Munich | 16 December 2024 | Duijs, Eric |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

EP 4 685 469 A1

## ANNEX TO THE EUROPEAN SEARCH REPORT
## ON EUROPEAN PATENT APPLICATION NO.

EP 24 19 0422

This annex lists the patent family members relating to the patent documents cited in the above-mentioned European search report.
The members are as contained in the European Patent Office EDP file on
The European Patent Office is in no way liable for these particulars which are merely given for the purpose of information.

16-12-2024

| Patent document cited in search report | | Publication date | Patent family member(s) | | Publication date |
|---|---|---|---|---|---|
| JP 2892084 | B2 | 17-05-1999 | JP 2892084 B2 | | 17-05-1999 |
| | | | JP H03255338 A | | 14-11-1991 |
| US 2023329290 | A1 | 19-10-2023 | EP 3984368 A1 | | 20-04-2022 |
| | | | US 2023329290 A1 | | 19-10-2023 |
| | | | WO 2022078989 A1 | | 21-04-2022 |
| US 2016341649 | A1 | 24-11-2016 | AU 2014364384 A1 | | 30-06-2016 |
| | | | CA 2933076 A1 | | 25-06-2015 |
| | | | CN 106030284 A | | 12-10-2016 |
| | | | EP 3084395 A1 | | 26-10-2016 |
| | | | KR 20160100986 A | | 24-08-2016 |
| | | | RU 2016129489 A | | 25-01-2018 |
| | | | US 2016341649 A1 | | 24-11-2016 |
| | | | WO 2015095667 A1 | | 25-06-2015 |
| CN 207571113 | U | 03-07-2018 | CN 207571113 U | | 03-07-2018 |
| | | | EP 3198269 A1 | | 02-08-2017 |
| | | | RU 2017114173 A | | 26-10-2018 |
| | | | US 2017248526 A1 | | 31-08-2017 |
| | | | WO 2016046603 A1 | | 31-03-2016 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2002054061 A1
- US 6365856 B1
- EP 0844026 A1
- US 3477568 A

**Non-patent literature cited in the description**

- **NAGEL-HELD, J.** ; **KAISER, L.** ; **LONGIN, C. F. H.** ; **HITZMANN, B.** Prediction of wheat quality parameters combining Raman, fluorescence, and near-infrared spectroscopy (NIRS). *Cereal Chem.*, 2022, vol. 99 (4), 830-842, https://doi.org/10.1002/cche.10540
- Principles of Fluorescence Spectroscopy. **LAKOWICZ, J.** Principles of Fluorescent Spectroscopy. 2006
- **OSBORNE, B.G.** Investigations into the use of near infrared reflectance spectroscopy for the quality assessment of wheat with respect to its potential for bread baking. *J. Sci. Food Agric.*, 1984, vol. 35 (1), 106-110
- **AHMAD, M. H.** ; **NACHE, M.** ; **WAFFENSCHMIDT, S.** ; **HITZMANN, B.** Characterization of faringographic kneading process for different types of wheat flours using fluorescence spectroscopy and chemometrics. *Food Control*, 2016, vol. 66, 44-52, https://doi.org/10.1016/j.foodcont.2016.01.029
- **RUBENTHALER, G.L.** ; **POMERANZ, Y.** Near-infrared reflectance spectra of hard red winter wheats varying widely in protein content and breadmaking potential. *Cereal Chem.*, 1987, vol. 64 (6), 407-411
- **DELWICHE, S.R.** ; **WEAVER, G.** Bread quality of wheat flour by near-infrared spectrophotometry: Feasibility of modelling. *J. Food Sci.*, 1994, vol. 59 (2), 410-415
- **BENGTSSON, S.** *NIR determination of flour baking properties*, 2002
- **SORVANIEMI, J.** ; **KINNUNEN, A.** ; **TSADOS, A.** ; **MÄLKKI, Y.** Using partial least squares regression and multiplicative scatter correction for FT-NIR data evaluation of wheat flours. *Lebensm. Wiss. Technol.*, 1993, vol. 26, 251-258
- **NAGEL-HELD, J.** ; **EL HASSOUNI, K.** ; **LONGIN, F.** ; **HITZMANN, B.** Spectroscopy-based prediction of 73 wheat quality parameters and insights for practical applications. *Cereal Chem.*, 2024, vol. 101, 144-165, https://doi.org/10.1002/cche.10732 en.wikipedia.org/wiki/Semolina

- **SACCHETTI, G.** ; **COCCOB. G.** ; **COCCOB, D.** ; **NERIA, L.** ; **MASTROCOLA, D.** Effect of semolina particle size on the cooking kinetics and quality of spaghetti. *Proc. Food Sci.*, 2011, vol. 1, 1740-1745, www.haverparticleanalysis.com/en/sieve-analysis/-ro-tapr-test-sieve-shaker/ www.retsch.com/products/sieving/sieve-shakers/as-200-control/function-features/ gkm-net.de/en/laboratory-air-jet-lab-sieves.html
- **HARELAND.** Evaluation of flour particle size distribution by laser diffraction, sieve analysis and near-infrared reflectance spectroscopy. *J. Cereal Sci.*, 1994, vol. 20 (2), 183-190
- **SHEWRY, P. R.** ; **HALFORD, N. G.** ; **BELTON, P. S.** ; **TATHAM, A. S.** The structure and properties of gluten: an elastic protein from wheat grain. *Phil. Trans. R. Soc. Lond.*, 2002, vol. 357, 133-142
- **CZAJA T** ; **SOBOTA A** ; **SZOSTAK R**. Quantification of Ash and Moisture in Wheat Flour by Raman Spectroscopy. *Foods*, 2020, vol. 9 (3), 280
- **JENSEN, S. A.** ; **MUNCK, L.** ; **MARTENS, H.** The Botanical Constituents of Wheat and Wheat Milling Fractions. Quantification by Autofluorescence. *Cereal Chem.*, 1982, vol. 59 (6), 477-484, https://www.cerealsgrains.org/publications/cc/backissues/1982/-Documents/chem59_477.pdf
- **JONES, C. R.** ; **HALTON, P.** ; **STEVENS, D. J.** The separation of flour into fractions of different protein contents by means of air classification. *J. Biochem. Microbiol. Technol. Eng.*, 1959, vol. 1 (1), 77-98, https://doi.org/10.1002/jbmte.390010108
- **YOUSSEF, M. M.** ; **ABO-DIEF, M. F.** Air Classification: Eco-Friendly Separation Technique in Food Technology- An Article. *Alexandria J. Food Sci. Technol.*, 2022, vol. 19 (1), 10-14, https://doi.org/10.21608/ajfs.2022.255779